# EUROPEAN PATENT SPECIFICATION

(11) **EP 0 711 305 B1**
(45) Date of publication and mention of the grant of the patent: **21.11.2001**
(21) Application number: 95906203.5
(22) Date of filing: 20.07.1994
(51) Int. Cl.: C07K 7/00, C07K 7/08, C07K 7/64, C12N 15/03, C12N 15/04, C12N 15/05, C12N 15/06, C12N 15/12

(54) **PROTEGRINS**
PROTEGRINE
PROTEGRINES

(30) Priority: 20.07.1993 US 93926; 26.07.1993 US 95769; 13.01.1994 US 182483; 17.05.1994 US 243879
(43) Date of publication of application: 15.05.1996
(73) Proprietor: UNIVERSITY OF CALIFORNIA, LOS ANGELES, Los Angeles, CA 90024-1406 (US)
(72) Inventor: LEHRER, Robert L., Santa Monica, CA 94043 (US); HARWIG, Sylvia S.L., Woodland Hills, CA 91364 (US); KOKRYAKOV, Vladimir N., Los Angeles, CA 90036 (US)
(74) Representative: Goldin, Douglas Michael
(86) International application number: PCT/US94/08305
(87) International publication number: WO 95/03325

(56) References cited:
- EP-A- 0 545 730
- US-A- 4 705 777
- C. ZHAO ET AL: "Identification of a new member of the protegrin family by cDNA cloning" FEBS LETTERS., vol. 346, 13 June 1994, AMSTERDAM NL, pages 285-288, XP002047193
- P. STORICI ET AL: "A novel cDNA sequence encoding a pig leukocite antimicrobial peptide with a cathelin-like pro-sequence" BIOCHEMICAL AND BIOPHYSICAL RESEARCH COMMUNICATIONS., vol. 196, no. 3, November 1993, ORLANDO, FL US, pages 1363-1368, XP002047194
- MIRGORODSKAYA P A ET AL: "ELECTROSPRAY IONIZATION TIME-OF-FLIGHT MASS SPECTROMETRY IN PROTEIN CHEMISTRY" ANALYTICAL CHEMISTRY, vol. 66, no. 1, 1 January 1994, pages 99-107, XP000426226
- LEHRER R L ET AL: "DEFENSINS: ENDOGENOUS ANTIBIOTIC PEPTIDES OF ANIMAL CELLS" CELL, vol. 64, no. 2, 25 January 1991, pages 229-230, XP000170612
- H. TAMAMURA ET AL.: "Antimicrobial activity and conformation of tachyplesin I and its analogs" CHEMICAL AND PHARMACEUTICAL BULLETIN., vol. 41, no. 5, 1993, TOKYO JP, pages 978-980, XP002047195
- THE JOURNAL OF BIOLOGICAL CHEMISTRY, Volume 260, Number 8, issued 25 April 1985, SELSTED et al.: "Primary structures of six antimicrobial peptides of rabbit peritoneal neutrophils", pages 4579-4584, see whole publication, especially page 4581.
- FEDERATION OF EUROPEAN BIOCHEMICAL SOCIETIES, Volume 327, Number 2, issued 26 July 1993, KOKRYAKOV et al.: "Protegrins: leukocyte antimicrobial peptides that combine features of corticostatic defensins and tachyplesins", pages 231-236, see whole publication.
- FEDERATION OF EUROPEAN BIOCHEMICAL SOCIETIES, Volume 330, Number 3, issued September 1993, MIRGORODSKAYA et al.: "Primary structure of three cationic peptides from porcine neutrophils: sequence determination by the combined usage of electrospray ionization mass spectrometry and Edman degradation", pages 339-342, see whole publication.

## Description

### Technical Field

The invention relates to the field of antibiotic peptides. In particular, the invention concerns short peptides, some of which are isolated from porcine leukocytes, that have a wide range of antimicrobial activities.

### Background Art

One of the defense mechanisms against infection by both animals and plants is the production of peptides that have antimicrobial and antiviral activity. Various classes of these peptides have been isolated from tissues both of plants and animals. One well known class of such peptides is the tachyplesins which were first isolated from the hemocytes of the horseshoe crab as described by Nakamura, T. *et al.* J Biol Chem (1988) 263:16709-16713. This article described the initial tachyplesin isolated, Tachyplesin I, from the Japanese species. Tachyplesin I is a 17-amino acid amidated peptide containing four cysteine residues providing two intramolecular cystine bonds. In a later article by this group, Miyata, T. *et al*. J Biochem (1989) 106:663-668, extends the studies to the American horseshoe crab and isolated a second tachyplesin, Tachyplesin II, consisting of 17 residues amidated at the C-terminus, also containing four cysteine residues and two intramolecular disulfide bonds. Two additional 18-mers, called polyphemusins, highly homologous to Tachyplesin II and containing the same positions for the four cysteine residues, were also isolated. Polyphemusin I and Polyphemusin II differ from each other only in the replacement of one arginine residue by a lysine. All of the peptides were described as having antifungal and antibacterial activity. A later article by Murakami, T. *et al.* Chemotherapy (1991) 37:327-334, describes the antiviral activity of the tachyplesins with respect to vesicular stomatus virus; Herpes Simplex Virus I & II, Adenovirus I, Reovirus II and Poliovirus I were resistant to inactivation by Tachyplesin I. Morimoto, M. *et al.* Chemotherapy (1991) 37:206-211, found that Tachyplesin I was inhibitory to Human Immunodeficiency Virus. This anti-HIV activity was found also to be possessed by a synthetic analog of Polyphemusin II as described by Nakashima, H. *et al.* Antimicrobial Agents and Chemotherapy (1992) 1249-1255. Antiviral peptides have also been found in rabbit leukocytes as reported by Lehrer, R.I. *et al.* J Virol (1985) 54:467-472.

Other important classes of cysteine-containing antimicrobial peptides include the defensins, β-defensins and insect defensins. The defensins are somewhat longer peptides characterized by six invariant cysteines and three intramolecular cystine disulfide bonds. Defensins were described by Lehrer, R.I. *et al.* Cell (1991) 64:229-230; Lehrer, R.I. *et al.* Ann Rev Immunol (1993) 11:105-128. A review of mammalian-derived defensins by Lehrer, R.I. *et al.* is found in Annual Review Immunol (1993) 11:105-128; three patents have issued on the defensins: U.S. 4,705,777; U.S. 4,659,692; and U.S. 4,543,252.
Defensins have been found in the polymorphonucleated neutrophils (PMN) of humans and of several other animals, as well as in rabbit pulmonary alveolar macrophages, and in murine small intestinal epithelial (Paneth) cells and in corresponding cells in humans.

β-Defensins are found in bovine respiratory epithelial cells, bovine granulocytes and avian leukocytes. See Selsted, M.E. *et al*. J Biol Chem (1993) 288:6641-6648 and Diamond, G. *et al.* Proc Natl Acad Sci (USA) (1991) 88:3952-3958. Insect defensins have been reported by Lambert, J. *et al.* Proc Natl Acad Sci (USA) (1989) 88:262-265.

Antifungal and antibacterial peptides and proteins have also been found in plants (Broekaert, W.F. *et al.* Biochemistry (1992) 31:4308-4314) as reviewed by Cornelissen, B.J.C. *et al.* Plant Physiol (1993) 101:709-712. Expression systems for the production of such peptides have been used to transform plants to protect the plants against such infection as described, for example, by Haln, R. *et al.* Nature (1993) 361:153-156.

The present invention provides a new class of antimicrobial and antiviral peptides, designated "protegrins" herein, representative members of which have been isolated from porcine leukocytes. These peptides are useful as antibacterial antiviral and antifungal agents in both plants and animals.

The isolation of the protegrin peptides of the invention was reported by the present applicants in a paper by Kokryakov, V.N. *et al.* FEBS (1993) 337:231-236 (July issue). A later publication of this group described the presence of a new protegrin, whose sequence, and that of its precursor, was deduced from its isolated cDNA clone. Zhao, C et al, FEBS Letters (1994) 346:285-288. An additional paper disclosing cationic peptides from porcine neutrophils was published by Mirgorodskaya, O.A. *et al.* FEBS (1993) 330:339-342 (September issue). Storici, P. *et al*. Biochem Biophys Res Comm (1993) 196:1363-1367, report the recovery of a DNA sequence which encodes a pig leukocyte antimicrobial peptide with a cathelin-like prosequence. The peptide is reported to be one of the protegrins disclosed hereinbelow.

The protegrins of the invention have also been found to bind to endotoxins -- i.e., the lipopolysaccharide (LPS) compositions derived from gram-negative bacteria which are believed responsible for gram-negative sepsis. This type of sepsis is an extremely common condition and is often fatal. Others have attempted to design and study proteins which bind LPS/endotoxin, and illustrative reports of these attempts appear in Rustici,
A. *et al.* Science (1993) 259:361-364; Matsuzaki, K. *et al.* Biochemistry (1993) 32:11704-11710; Hoess, A. *et al.* EMBO J (1993) 12:3351-3356; and Elsbach, P. *et al.* Current *Opinion* in Immunology (1993) 5:103-107. The protegrins of the present invention provide additional compounds which are capable of inactivating of LPS and ameliorating its effects.

In addition to the foregoing, the protegrins of the invention are effective in inhibiting the growth of organisms that are associated with sexually transmitted diseases. It is estimated that 14 million people worldwide are infected with HIV and that millions of women sustain pelvic inflammatory disease each year. *Chlamydia trachomatis* and *Neisseria gonorrhoeae* cause over half of this inflammatory disease although *E*. *coli, Mycoplasma hominis* and other infectious microorganisms can also be responsible. Pathogens include viral, bacterial, fungal and protozoan pathogens. It is especially important that the antibiotics used to combat these infections be effective under physiological conditions. The protegrins of the present invention offer these properties.

### Disclosure of the Invention

In one embodiment, the invention is directed to peptides of 16-18 amino acid residues characterized by four invariant cysteines and either by a characteristic pattern of basic and hydrophobic amino acids and/or being isolatable from animal leukocytes using the method described herein. In a second embodiment, the invention is directed to the above peptides wherein 1-4 of these cysteines is replaced by a hydrophobic or small amino acid. These peptides can be produced synthetically and some can be produced recombinantly or can be isolated from their native sources and purified for use as preservatives or in pharmaceutical compositions in treating or preventing infection in animals. Alternatively, the peptides can be formulated into compositions which can be applied to plants to protect them against viral or microbial infection. In still another approach, the DNA encoding the peptides can be expressed *in situ,* in animals or preferably in plants, to combat infections. The peptides are also useful as standards in antimicrobial assays and in binding endotoxins

Accordingly, in one aspect, the invention is directed to peptides of the formula:

A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1);

and the N-terminal acylated and/or C-terminal amidated or esterified forms thereof, which is either in the optionally -SH stabilized linear or in a cystine-bridged form
wherein each of A₁ and A₉ is independently a basic amino acid;
each of A₂ and A₃ is independently a small amino acid;
each of A₅, A₇, A₁₂, A₁₄ and A₁₆ is independently a hydrophobic amino acid;
each of A₄ and A₁₀ is independently a basic or a small amino acid;
A₁₁ is a basic or a hydrophobic amino acid;
A₁₇ is not present or, if present, is a small amino acid;
A₁₈ is not present or, if present, is a basic amino acid, or a
modified form of formula (1) and the N-terminal acylated and/or C-terminal amidated or esterified forms thereof wherein each of 1-4 cysteines is independently replaced by a hydrophobic amino acid or a small amino acid;
with the proviso that if said compound is of the formula 
RGGRLCYCRRRFCVCVGR,
RGGRLCYCRRRFCICV,
 
RGGGLCYCRRRFCVCVGR, or
RGGRLCYCRGWICFCVGR
 in the amidated and di-cystine-bridged form, said compound is purified and isolated.

In another aspect, the invention comprises a purified and isolated peptide of the formula:

A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1a);

and the amidated or esterified and/or N-terminal acylated forms thereof, including the optionally SH-stabilized linear and the cyclic forms thereof
wherein A₁₋₅, A₇, A₉₋₁₂ and A₁₄ and A₁₆, and, if present, A₁₇ and A₁₈ (i.e. Aₙ), represent amino acid residues
which peptides are isolatable from animal leukocytes by the methods similar to those described herein.

In still other aspects, the invention is directed to recombinant materials useful for the production of the peptides of the invention. The invention is also directed to pharmaceutical compositions and compositions for application to plants containing the peptides of the invention as active ingredients. The invention is also directed to antibodies specific for these peptides.

The compounds of the invention may be used as standards in antimicrobial assays. The compounds may also be used as antimicrobials in solutions useful in eye care, such as contact lens solutions, and in topical or other pharmaceutical compositions for treatment of sexually transmitted diseases (STDs). The invention compounds may also be used as preservatives for foods or other perishables. As the invention peptides can inactivate endotoxins, the invention is also directed to inactivation of endotoxins using the compounds of the invention.

### Brief Description of the Drawings

Figure 1 shows the elution pattern of a concentrate of the ultrafiltrate of porcine leukocytes applied to a Biogel P10 column.

Figure 2 shoes the antibacterial activity of the P10 fractions obtained from elution of the column described in Figure 1.

Figure 3 shows an elution pattern obtained when fractions 76-78 from the Biogel P10 column of Figure 1 is applied to HPLC.

Figure 4 shows the antimicrobial activity of the purified porcine protegrins of the invention:
Figure 4a shows antibacterial activity against *E.coli*;
Figure 4b shows antibacterial activity against *Listeria monocytogenes*;
Figure 4c shows antifungal activity against *Candida albicans*;
Figure 4d shows antibacterial activity against *S. aureus*;
Figure 4e shows antibacterial activity against *K. pneumoneae.*

Figure 5 shows the effect of various test conditions on antimicrobial activity:
Figure 5a shows activity against *Candida albicans* in 100 *µ*M NaCl;
Figure 5b shows activity against *E*. *Coli* in 100 *µ*M NaCl;
Figure 5c shows activity against *Candida albicans* in 90% fetal calf serum.

Figure 6 shows the antimicrobial activity of the linear forms of the protegrins under various test conditions:
Figure 6a shows the activity against *E*. *coli* in 10 mM phosphate-citrate buffer, pH 6.5;
Figure 6b shows the activity against *E*. *coli* in the same buffer with 100 mM NaCl;
Figure 6c shows the activity against *L. monocytogenes* in the buffer of Figures 6a-6b;
Figure 6d shows the activity against *L. monocytogenes* in the same buffer with the addition of 100 mM NaCl;
Figure 6e shows the activity against *C. albicans* in the presence of 10 mM phosphate; and
Figure 6f shows the activity against *C. albicans* in the presence of 10 mM phosphate plus 100 mM NaCl.

Figure 7 shows a composite of cDNA encoding the precursors of PG-1, PG-2, PG-3 and PG-4.

Figure 8 shows the amino acid sequences of the protegrins of Figure 7.

Figures 9a-9d show the effects of various protegrins against various target microbes.

### Modes of Carrying Out the Invention

The peptides of the invention are described by the formula:

A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈), (1)

and its defined modified forms. Those peptides which occur in nature must be in purified and isolated form.

The designation Aₙ in each case represents an amino acid at the specified position in the peptide. As A₁₇ and A₁₈ may or may not be present, the peptides of the invention contain either 16, 17 or 18 amino acids. The positions of the cysteine residues, shown as C in Formula (1), are invariant in the peptides of the invention; however, in the modified forms of the peptides of Formula (1), also included within the scope of the invention, 1-4 of these cysteines may be replaced by a hydrophobic or small amino acid.

The amino terminus of the peptide may be in the free amino form or may be acylated by a group of the formula RCO-, wherein R represents a hydrocarbyl group of 1-6C. The hydrocarbyl group is saturated or unsaturated and is typically, for example, methyl, ethyl, i-propyl, t-butyl, n-pentyl, cyclohexyl, cyclohexene-2-yl, hexene-3-yl, hexyne-4-yl, and the like.

The C-terminus of the peptides of the invention may be in the form of the underivatized carboxyl group, either as the free acid or an acceptable salt, such as the potassium, sodium, calcium, magnesium, or other salt of an inorganic ion or of an organic ion such as caffeine. The carboxyl terminus may also be derivatized by formation of an ester with an alcohol of the formula ROH, or may be amidated by an amine of the formula NH₃, or RNH₂, or R₂NH, wherein each R is independently hydrocarbyl of 1-6C as defined above. Amidated forms of the peptides wherein the C-terminus has the formula CONH₂ are preferred.

As the peptides of the invention contain substantial numbers of basic amino acids, the peptides of the invention may be supplied in the form of the acid addition salts. Typical acid addition salts include those of inorganic ions such as chloride, bromide, iodide, fluoride or the like, sulfate, nitrate, or phosphate, or may be salts of organic anions such as acetate, formate, benzoate and the like. The acceptability of each of such salts is dependent on the intended use, as is commonly understood.

The peptides of the invention that contain at least two cysteines may be in straight-chain or cyclic form. The straight-chain forms are convertible to the cyclic forms, and *vice versa.* Methods for forming disulfide bonds to create the cyclic peptides are well known in the art, as are methods to reduce disulfides to form the linear compounds. The linear compounds can be stabilized by addition of a suitable alkylating agent such as iodoacetamide.

The cyclic forms are the result of the formation of cystine linkages among all or some of the four invariant cysteine residues. Cyclic forms of the invention include all possible permutations of cystine bond formation; if the cysteines are numbered in order of their occurrence starting at the N-terminus as C₆, C₈, C₁₃ and C₁₅, these permutations include:
C₆-C₈;
C₆-C₁₃;
C₆-C₁₅ ;
C₈-C₁₃;
C₈-C₁₅;
C₁₃-C₁₅;
C₆-C₈, C₁₃-C₁₅;
C₆-C₁₃, C₈-C₁₅; and
C₆-C₁₅, C₈-C₁₃.

In the modified forms of the peptides, where 1-4 cysteines are replaced, similar permutations are available when 2-3 cysteines are present.

The native forms of the protegrins contain two cystine bonds are between the cysteine at position 6 and the cysteine at position 15 and the other between the cysteine at position 8 and the cysteine at position 13. Accordingly, in those embodiments having two cystine linkages, the C₆-C₁₅, C₈-C₁₃ form is preferred. However, it has been found by the present applicants that forms of the protegrins containing only one cystine linkage are active and easily prepared. Preferred among embodiments having only one cystine linkage are those represented by C₆-C₁₅ alone and by C₈-C₁₃ alone.

As the linearalized forms of the native cyclic peptides have valuable activities, even when chemically stabilized to preserve the sulfhydryl form of cysteine for example, by reaction with iodoacetamide, the compounds of the invention also include linearalized forms which are stabilized with suitable reagents. As defined herein, "SH-stabilized" forms of the peptides of the invention contain sulfhydryl groups reacted with standard reagents to prevent reformation into disulfide linkages.

An alternative approach to providing linear forms of the protegrins of the invention comprises use of the modified form of the peptides where cysteine residues are replaced by amino acids which do not form cystine linkages.

The amino acids denoted by Aₙ may be those encoded by the gene or analogs thereof, and may also be the D-isomers thereof. One preferred embodiment of the peptides of the invention is that form wherein all of the residues are in the D-configuration thus conferring resistance to protease activity while retaining antimicrobial or antiviral properties. The resulting protegrins are themselves enantiomers of the native L-amino acid-containing forms.

The amino acid notations used herein are conventional and are as follows:

| Amino Acid | One-Letter Symbol | Three-letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

The amino acids not encoded genetically are abbreviated as indicated in the discussion below.

In the specific peptides shown in the present application, the L-form of any amino acid residue having an optical isomer is intended unless the D-form is expressly indicated by a dagger superscript (^{†}).

The compounds of the invention are peptides which are partially defined in terms of amino acid residues of designated classes. Amino acid residues can be generally subclassified into major subclasses as follows:

Acidic: The residue has a negative charge due to loss of H ion at physiological pH and the residue is attracted by aqueous solution so as to seek the surface positions in the conformation of a peptide in which it is contained when the peptide is in aqueous medium at physiological pH.

Basic: The residue has a positive charge due to association with H ion at physiological pH and the residue is attracted by aqueous solution so as to seek the surface positions in the conformation of a peptide in which it is contained when the peptide is in aqueous medium at physiological pH.

Hydrophobic: The residues are not charged at physiological pH and the residue is repelled by aqueous solution so as to seek the inner positions in the conformation of a peptide in which it is contained when the peptide is in aqueous medium.

Neutral/polar: The residues are not charged at physiological pH, but the residue is not sufficiently repelled by aqueous solutions so that it would seek inner positions in the conformation of a peptide in which it is contained when the peptide is in aqueous medium.

This description also characterizes certain amino acids as "small" since their side chains are not sufficiently large, even if polar groups are lacking, to confer hydrophobicity. "Small" amino acids are those with four carbons or less when at least one polar group is on the side chain and three carbons or less when not.

It is understood, of course, that in a statistical collection of individual residue molecules some molecules will be charged, and some not, and there will be an attraction for or repulsion from an aqueous medium to a greater or lesser extent. To fit the definition of "charged," a significant percentage (at least approximately 25%) of the individual molecules are charged at physiological pH. The degree of attraction or repulsion required for classification as polar or nonpolar is arbitrary and, therefore, amino acids specifically contemplated by the invention have been classified as one or the other. Most amino acids not specifically named can be classified on the basis of known behavior.

Amino acid residues can be further subclassified as cyclic or noncyclic, and aromatic or nonaromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always nonaromatic.

For the naturally occurring protein amino acids, subclassification according to the foregoing scheme is as follows.
Acidic: Aspartic acid and Glutamic acid;
Basic:
   Noncyclic: Arginine, Lysine;
   Cyclic: Histidine;
Small: Glycine, Serine, Alanine, Threonine;
Polar/large: Asparagine, Glutamine;
Hydrophobic: Tyrosine, Valine, Isoleucine, Leucine, Methionine, Phenylalanine, Tryptophan.

The gene-encoded secondary amino acid proline is a special case due to its known effects on the secondary conformation of peptide chains, and is not, therefore, included in a group. Cysteine residues are also not included in these classifications since their capacity to form disulfide bonds to provide secondary structure is critical in the compounds of the present invention.

Certain commonly encountered amino acids, which are not encoded by the genetic code, include, for example, beta-alanine (beta-Ala), or other omega-amino acids, such as 3-aminopropionic, 2,3-diaminopropionic (2,3-diaP), 4-aminobutyric and so forth, alpha-aminisobutyric acid (Aib), sarcosine (Sar), ornithine (Orn), citrulline (Cit), t-butylalanine (t-BuA), t-butylglycine (t-BuG), N-methylisoleucine (N-MeIle), phenylglycine (Phg), and cyclohexylalanine (Cha), norleucine (Nle), 2-naphthylalanine (2-Nal); 1,2,3,4-tetrahydroisoquinoline-3-carboxylic acid (Tic); β-2-thienylalanine (Thi); methionine sulfoxide (MSO); and homoarginine (Har). These also fall conveniently into particular categories.

Based on the above definitions,
Sar, beta-Ala, 2,3-diaP and Aib are small;
t-BuA, t-BuG, N-MeIle, Nle, Mvl, Cha, Phg, Nal, Thi and Tic are hydrophobic;
Orn and Har are basic;
Cit, Acetyl Lys, and MSO are neutral/polar.

The various omega-amino acids are classified according to size as small (beta-Ala and 3-aminopropionic) or as large and hydrophobic (all others).

Other amino acid substitutions of those encoded in the gene can also be included in peptide compounds within the scope of the invention and can be classified within this general scheme according to their structure.

In all of the peptides of the invention, one or more amide linkages (-CO-NH-) may optionally be replaced with another linkage which is an isostere such as -CH₂NH-, -CH₂S-, -CH₂CH₂, -CH=CH- (cis and trans), -COCH₂-, -CH(OH)CH₂- and -CH₂SO-. This replacement can be made by methods known in the art. The following references describe preparation of peptide analogs which include these alternative-linking moieties: Spatola, A.F., Vega Data (March 1983), Vol. 1, Issue 3, "Peptide Backbone Modifications" (general review); Spatola, A.F., in "Chemistry and Biochemistry of Amino Acids Peptides and Proteins," B. Weinstein, eds., Marcel Dekker, New York, p. 267 (1983) (general review); Morley, J.S., Trends Pharm Sci (1980) pp. 463-468 (general review); Hudson, D., et al., Int J Pept Prot Res (1979) 14:177-185 (-CH₂NH-, -CH₂CH₂-); Spatola, A.F., et al., Life Sci (1986) 38:1243-1249 (-CH₂-S); Hann, M.M., J Chem Soc Perkin Trans I (1982) 307-314 (-CH-CH-, cis and trans); Almquist, R.G., et al., J Med Chem (1980) 23:1392-1398 (-COCH₂-); Jennings-White, C., et al., Tetrahedron Lett (1982) 23:2533 (-COCH₂-); Szelke, M., et al., European Application EP 45665 (1982) CA:97:39405 (1982) (-CH(OH)CH₂-) ; Holladay, M.W., et al., Tetrahedron Lett (1983) 24:4401-4404 (-C(OH)CH₂-) ; and Hruby, V.J., Life Sci (1982) 31:189-199 (-CH₂-S-).

The compounds of Formula (1) are generally defined as

A₁-A₂-A₃-A₄-A₅-C₆-A₇-C₇-A₉-A₁₀-A₁₁-A₁₂-C₁₃-A₁₄-C₁₅-A₁₆-(A₁₇-A₁₈) (1)

and the N-terminal acylated and/or C-terminal amidated or esterified forms thereof, which is either in the optionally -SH stabilized linear or in a cystine-bridged form
wherein each of A₁ and A₉ is independently a basic amino acid;
each of A₂ and A₃ is independently a small amino acid;
each of A₅, A₇, A₁₂, A₁₄ and A₁₆ is independently a hydrophobic amino acid;
each of A₄ and A₁₀ is independently a basic or a small amino acid;
A₁₁ is a basic or a hydrophobic amino acid; A₁₇ is not present or, if present, is a small amino acid;
A₁₈ is not present or, if present, is a basic amino acid, or a modified form of formula (1) and the N-terminal acylated and/or C-terminal amidated or esterified forms thereof wherein each of 1-4 cysteines is independently replaced by a hydrophobic amino acid or a small amino acid.
with the proviso that if said compound is of the formula 
RGGRLCYCRRRFCVCVGR,
RGGRLCYCRRRFCICV,
RGGGLCYCRRRFCVCVGR, or
RGGRLCYCRGWICFCVGR
 in the amidated and di-cystine-bridged form, said compound is purified and isolated.

In preferred embodiments of the compounds of the invention, each of A₁ and A₉ is independently selected from the group consisting of R, K and Har; more preferably, both A₁ and A₉ are R.

In another class of preferred embodiments, each of A₂ and A₃ is independently selected from the group consisting of G, A, S and T; more preferably, A₂ and A₃ are G.

In another set of preferred embodiments, A₄ is selected from the group consisting of R, K, Har, G, A, S and T; more preferably, A₄ is R or G.

In another set of preferred embodiments, each of A₅, A₁₄ and A₁₆ is independently selected independently from the group consisting of I, V, L, Nle and F; preferably I, V, L and F.

In another set of preferred embodiments, each of A₇ and A₁₂ is independently selected from the group consisting of I, V, L, W, Y and F; preferably A₇ is Y and A₁₂ is I or F.

In another set of preferred embodiments, each of A₁₀ and A₁₁ is independently R, G or W.

A₁₇, when present, is preferably G, A, S or T, most preferably G;

A₁₈, when present, is preferably R, K or Har, most preferably R.

As described above, the compounds of Formula (1) are either in cyclic or noncyclic (linearalized) form or may be modified wherein 1-4 of the cysteines is replaced by a small amino acid residue or a basic amino acid residue. If the linearalized forms of the compound of Formula (1) are prepared, or if linearalized forms of those modified peptides which contain at least two cysteines are prepared, it is preferred that the sulfhydryl groups be stabilized by addition of a suitable reagent. Preferred embodiments for the hydrophobic amino acid to replace cysteine residues are I, V, L and NLe, preferably I, V or L. Preferred small amino acids to replace the cysteine residues include G, A, S and T, most preferably G.

In an alternative embodiment, the peptides of the invention are defined as described by Formula (1), but wherein the definitions of Aₙ in each case are determined by the isolatability of the peptide from animal leukocytes by the invention method. The invention method comprises the steps of providing an ultrafiltrate of a lysate of animal leukocytes and isolating peptides of 16-18 amino acids. These peptides can further be defined by the ability of DNA encoding them to hybridize under stringent conditions to DNA encoding the peptides exemplified as PG-1, PG-2, PG-3, and PG-4 herein.

Particularly preferred compounds of the invention are:

### Unmodified forms

PG-1: R-G-G-R-L-C-Y-C-R-R-R-F-C-V-C-V-G-R

PG-2: R-G-G-R-L-C-Y-C-R-R-R-F-C-I-C-V
PG-3: R-G-G-G-L-C-Y-C-R-R-R-F-C-V-C-V-G-R
PG-4: R-G-G-R-L-C-Y-C-R-G-W-I-C-F-C-V-G-R
R-G-G-R-L-C-Y-C-R-R-R-F-C-V-C-V
K-G-G-R-L-C-Y-C-R-R-R-F-C-V-C-V
R-G-G-Har-L-C-Y-C-R-R-R-F-C-V-C-V
R-G-G-Har-L-C-Y-C-Har-R-R-F-C-V-C-V-G-R
R-G-G-R-V-C-Y-C-R-Har-R-F-C-V-C-V-G-R
R-G-G-R-L-C-Y-C-R-K-K-W-C-V-C-V-G-R
R-G-G-R-L-C-Y-C-R-Har-R-Y-C-V-C-V-G-R
R-G-S-G-L-C-Y-C-R-R-K-W-C-V-C-V-G-R
R-A-T-R-I-C-F-C-R-R-R-F-C-V-C-V-G-R
R-G-G-K-V-C-Y-C-R-Har-R-F-C-V-C-V-G-R
R-A-T-R-I-C-F-C-R†-R-R-F-C-V-C-V-G-R†
R-G-G-K-V-C-Y-C-R-Har†-R-F-C-V-C-V-G-R
PG-1: R-G-G-R-L-C-Y-C-R-R-R-F-C-V-C-V-G-R (all †)
PG-2: R-G-G-R-L-C-Y-C-R-R-R-F-C-I-C-V (all †)
PG-3: R-G-G-G-L-C-Y-C-R-R-R-F-C-V-C-V-G-R (all †)
PG-4: R-G-G-R-L-C-Y-C-R-G-W-I-C-F-C-V-G-R (all †)

both the linear and mono- and bicyclic forms thereof, and including the N-terminal acylated and C-terminal amidated forms;

### Modified forms

R-G-G-R-L-V-Y-C-R-R-R-F-C-V-C-V-G-R
R-G-G-R-L-G-Y-C-R-R-R-F-C-I-C-V
R-G-G-G-L-C-Y-G-R-R-R-F-C-V-C-V-G-R
R-G-G-R-L-G-Y-G-R-R-R-F-G-V-C-V
K-G-G-R-L-V-Y-V-R-R-R-F-I-V-C-V
R-G-G-Har-L-C-Y-C-R-R-R-F-C-V-G-V
R-G-G-Har-L-C-Y-C-Har-R-R-F-C-V-L-V-G-R
R-G-G-R-V-C-Y-V-R-Har-R-F-L-V-G-V-G-R
R-G-G-R-L-C-Y-S-R-K-K-W-C-V-S-V-G-R
R-G-G-R-L-C-Y-C-R-Har-R-Y-S-V-V-V-G-R
R-G-S-G-L-S-Y-C-R-R-K-W-G-V-C-V-G-R
R-A-T-R-I-S-F-S-R-R-R-F-S-V-S-V-G-R
R-G-G-K-V-C-Y-G-R-Har-R-F-S-V-C-V-G-R
R-A-T-R-I-V-F-C-R†-R-R-F-G-V-C-V-G-R†

R-G-G-K-V-C-Y-L-R-Har†-R-F-L-V-C-V-G-R

both the linear and cyclic (where possible) forms thereof, and including the N-terminal acylated and C-terminal amidated forms.

### Preparation of the Invention Compounds

The invention compounds, often designated herein "protegrins" are essentially peptide backbones which may be modified at the N- or C-terminus and also may contain one or two cystine disulfide linkages. The peptides may first be synthesized in noncyclized form. These peptides may then be converted to the cyclic peptides if desired by standard methods of cystine bond formation. As applied to the protegrins herein, "cyclic forms" refers to those forms which contain cyclic portions by virtue of the formation of disulfide linkages between cysteine residues in the peptide. If the straight-chain forms are preferred, it is preferable to stabilize the sulfhydryl groups for any peptides of the invention which contain two or more cysteine residues.

Standard methods of synthesis of peptides the size of protegrins are known. Most commonly used currently are solid phase synthesis techniques; indeed, automated equipment for systematically constructing peptide chains can be purchased. Solution phase synthesis can also be used but is considerably less convenient. When synthesized using these standard techniques, amino acids not encoded by the gene and D-enantiomers can be employed in the synthesis. Thus, one very practical way to obtain the compounds of the invention is to employ these standard chemical synthesis techniques.

In addition to providing the peptide backbone, the N- and/or C-terminus can be derivatized, again using conventional chemical techniques. The compounds of the invention may optionally contain an acyl group, preferably an acetyl group at the amino terminus. Methods for acetylating or, more generally, acylating, the free amino group at the N-terminus are generally known in the art; in addition, the N-terminal amino acid may be supplied in the synthesis in acylated form.

At the carboxy terminus, the carboxyl group may, of course, be present in the form of a salt; in the case of pharmaceutical compositions this will be a pharmaceutically acceptable salt. Suitable salts include those formed with inorganic ions such as NH₄⁺, Na⁺, K⁺, Mg⁺⁺, Ca⁺⁺, and the like as well as salts formed with organic cations such as those of caffeine and other highly substituted amines. The carboxy terminus may also be esterified using alcohols of the formula ROH wherein R is hydrocarbyl (1-6C) as defined above. Similarly, the carboxy terminus may be amidated so as to have the formula -CONH₂, -CONHR, or -CONR₂, wherein each R is independently hydrocarbyl (1-6C) as herein defined. Techniques for esterification and amidation as well as neutralizing in the presence of base to form salts are all standard organic chemical techniques.

If the peptides of the invention are prepared under physiological conditions, the side-chain amino groups of the basic amino acids will be in the form of the relevant acid addition salts.

Formation of disulfide linkages, if desired, is conducted in the presence of mild oxidizing agents. Chemical oxidizing agents may be used, or the compounds may simply be exposed to the oxygen of the air to effect these linkages. Various methods are known in the art. Processes useful for disulfide bond formation have been described by Tam, J.P. et al., Synthesis (1979) 955-957; Stewart, J.M. et al, "Solid Phase Peptide Synthesis" 2d Ed. Pierce Chemical Company Rockford, IL (1984); Ahmed A.K. et al., J Biol Chem (1975) 250:8477-8482 and Pennington M.W. et al., Peptides 1990, E. Giralt et al., ESCOM Leiden, The Netherlands (1991) 164-166. An additional alternative is described by Kamber, B. et al., Helv Chim Acta (1980) 63:899-915. A method conducted on solid supports is described by Albericio Int J Pept Protein Res (1985) 26:92-97.

A particularly preferred method is solution oxidation using molecular oxygen. This method has been used by the inventors herein to refold synthetic PG-1, PG-3 in its amide or acid forms, *enantio*PG-1 and the two unisulfide PG-1 compounds (C₆-C₁₅ and C₈-C₁₃). Recoveries are as high as 30%.

If the peptide backbone is comprised entirely of gene-encoded amino acids, or if some portion of it is so composed, the peptide or the relevant portion may also be synthesized using recombinant DNA techniques. The DNA encoding the peptides of the invention may itself be synthesized using commercially available equipment; codon choice can be integrated into the synthesis depending on the nature of the host. Alternatively, although less convenient, the DNA can be obtained, at least initially, by screening a cDHA library prepared from porcine leukocytes using probes or PCR primers based on the sequences of the protegrins described herein.
This results in recovery of the naturally occurring sequence encoding the protegrins of the invention. Obtention of this native sequence is significant for purposes other than the synthesis of the protegrins per se; the availability of the naturally occurring sequences provides a useful probe to obtain corresponding DNA encoding protegrins of other species. Thus, cDNA libraries, for example, of leukocytes derived from other animals can be screened using the native DNA, preferably under conditions of high stringency. High stringency is as defined by Maniatis, *et al.* Molecular Cloning: a Laboratory Manual 2nd Ed, Cold Spring Harbor Laboratory Press (1989), the relevant portions of which are incorporated herein by reference. This procedure also permits recovery of allelic variants of these peptides from the same species.

Alternatively, the protegrins can be prepared by isolation from leukocytes of a desired species using techniques similar to those disclosed herein for the isolation of porcine protegrins. In general, these techniques involve preparing a lysate of a leukocyte preparation, ultrafiltering the supernatant of the clarified lysate and recovering the ultrafiltrate. The ultrafiltrate is then subjected to chromatographic separation. The location of fragments having antimicrobial and antiviral activity corresponding to protegrins can be assessed using criteria of molecular weight and assaying the fractions for the desired activities as described herein. The native forms of these peptides are believed to be the cyclic forms; if desired, the linearalized forms can be prepared by treating the peptides with reducing agents and stabilizing the sulfhydryl groups that result.

Isolated and recombinantly produced forms of the protegrins may require subsequent derivatization to modify the N- and/or C-terminus and, depending on the isolation procedure, to effect the formation of cystine bonds as described hereinabove. Depending on the host organism used for recombinant production and the animal source from which the protein is isolated, some or all of these conversions may already have been effected.

For recombinant production, the DNA encoding the protegrins of the invention is included in an expression system which places these coding sequences under control of a suitable promoter and other control sequences compatible with an intended host cell. Types of host cells available span almost the entire range of the plant and animal kingdoms. Thus, the protegrins of the invention could be produced in bacteria or yeast (to the extent that they can be produced in a nontoxic or refractile form or utilize resistant strains) as well as in animal cells, insect cells and plant cells. Indeed, modified plant cells can be used to regenerate plants containing the relevant expression systems so that the resulting transgenic plant is capable of self protection vis-à-vis these infective agents.

The protegrins of the invention can be produced in a form that will result in their secretion from the host cell by fusing to the DNA encoding the protegrin, a DNA encoding a suitable signal peptide, or may be produced intracellularly. They may also be produced as fusion proteins with additional amino acid sequence which may or may not need to be subsequently removed prior to the use of these compounds as antimicrobials or antivirals.

Thus, the protegrins of the invention can be produced in a variety of modalities including chemical synthesis, recombinant production, isolation from natural sources, or some combination of these techniques.

Those members of the protegrin class which occur naturally are supplied in purified and isolated form. By "purified and isolated" is meant free from the environment in which the peptide normally occurs (in the case of such naturally occurring peptides) and in a form where it can be used practically. Thus, "purified and isolated" form means that the peptide is substantially pure, i.e., more than 90% pure, preferably more than 95% pure and more preferably more than 99% pure or is in a completely different context such as that of a pharmaceutical preparation.

### Antibodies

Antibodies to the protegrins of the invention may also be produced using standard immunological techniques for production of polyclonal antisera and, if desired, immortalizing the antibody-producing cells of the immunized host for sources of monoclonal antibody production. Techniques for producing antibodies to any substance of interest are well known. It may be necessary to enhance the immunogenicity of the substance, particularly as here, where the material is only a short peptide, by coupling the hapten to a carrier. Suitable carriers for this purpose include substances which do not themselves produce an immune response in the mammal to be administered the hapten-carrier conjugate. Common carriers used include keyhole limpet hemocyanin (KLH), diphtheria toxoid, serum albumin, and the viral coat protein of rotavirus, VP6. Coupling of the hapten to the carrier is effected by standard techniques such as contacting the carrier with the peptide in the presence of a dehydrating agent such as dicyclohexylcarbodiimide or through the use of linkers such as those available through Pierce Chemical Company, Chicago, IL.

The protegrins of the invention in immunogenic form are then injected into a suitable mammalian host and antibody titers in the serum are monitored. It should be noted, however, that some forms of the protegrins require modification before they are able to raise antibodies, due to their resistance to antigen processing. For example, the native form of PG-1, containing two cystine bridges is nonimmunogenic when administered without coupling to a larger carrier and was a poor immunogen even in the presence of potent adjuvants and when coupled through glutaraldehyde or to KLH. Applicants believe this to be due to its resistance to attack by leukocyte serine proteases (human PMN elastase and cathepsin G) as well as to attack by an aspartic protease (pepsin) that resembles several macrophage cathepsins. The lack of immunogenicity may therefore result from resistance to processing to a linear form that can fit in the antigen-presenting pocket of the presenting cell. Immunogenecity of these forms of the protegrins can be enhanced by cleaving the disulfide bonds.

Polyclonal antisera may be harvested when titers are sufficiently high. Alternatively, antibody-producing cells of the host such as spleen cells or peripheral blood lymphocytes may be harvested and immortalized. The immortalized cells are then cloned as individual colonies and screened for the production of the desired monoclonal antibodies.

The antibodies of the invention are, of course, useful in immunoassays for determining the amount or presence of the protegrins. Such assays are essential in quality controlled production of compositions containing the protegrins of the invention. In addition, the antibodies can be used to assess the efficacy of recombinant production of the protegrins, as well as screening expression libraries for the presence of protegrin encoding genes.

### Compositions Containing the Protegrins and Their Use

The protegrins of the invention are effective in inactivating a wide range of microbial and viral targets, including gram-positive and gram-negative bacteria, yeast, protozoa and certain strains of virus. Accordingly, they can be used in disinfectant compositions and as preservatives for materials such as foodstuffs, cosmetics, medicaments, or other materials containing nutrients for organisms. For use in such contexts, the protegrins are supplied either as a single protegrin, in admixture with several other protegrins, or in admixture with additional antimicrobial agents. In general, as these are preservatives in this context, they are usually present in relatively low amounts, of less than 5%, by weight of the total composition, more preferably less than 1%, still more preferably less than 0.1%.

The peptides of the invention are also useful as standards in antimicrobial assays and in assays for determination of capability of test compounds to bind to endotoxins such as lipopolysaccharides.

For use as antimicrobials or antivirals for treatment of animal subjects, the protegrins of the invention can be formulated as pharmaceutical or veterinary compositions. Depending on the subject to be treated, the mode of administration, and the type of treatment desired -- e.g., prevention, prophylaxis, therapy; the protegrins are formulated in ways consonant with these parameters. A summary of such techniques is found in Remington's Pharmaceutical Sciences, latest edition, Mack Publishing Co., Easton, PA.

The protegrins are particularly attractive as an active ingredients pharmaceutical compositions useful in treatment of sexually transmitted diseases, including those caused by *Chlamydia trachomatis, Treponema pallidum*, *Neisseria gonorrhoeae, Trichomonas vaginalis*, Herpes simplex type 2 and HIV. Topical formulations are preferred and include creams, salves, oils, powders, gels and the like. Suitable topical excipient are well known in the art and can be adapted for particular uses by those of ordinary skill.

In general, for use in treatment or prophylaxis of STDs, the protegrins of the invention may be used alone or in combination with other antibiotics such as erythromycin, tetracycline, macrolides, for example azithromycin and the cephalosporins. Depending on the mode of administration, the protegrins will be formulated into suitable compositions to permit facile delivery to the affected areas. The protegrins may be used in forms containing one or two disulfide bridges or may be in linear form. In addition, use of the enantiomeric forms containing all D-amino acids may confer advantages such as resistance to those proteases, such as trypsin and chymotrypsin, to which the protegrins containing L-amino acids are less resistant.

The protegrins of the invention can be administered singly or as mixtures of several protegrins or in combination with other pharmaceutically active components. The formulations may be prepared in a manner suitable for systemic administration or topical or local administration. Systemic formulations include those designed for injection (e.g., intramuscular, intravenous or subcutaneous injection) or may be prepared for transdermal, transmucosal, or oral administration. The formulation will generally include a diluent as well as, in some cases, adjuvants, buffers, preservatives and the like. The protegrins can be administered also in liposomal compositions or as microemulsions.

If administration is to be oral, the protegrins of the invention must be protected from degradation in the stomach using a suitable enteric coating. This may be avoided to some extent by utilizing amino acids in the D-configuration, thus providing resistance to protease. However, the peptide is still susceptible to hydrolysis due to the acidic conditions of the stomach; thus, some degree of enteric coating may still be required.

As described in the examples below, the peptides of the invention retain their activity against microbes in the context of borate solutions that are commonly used in eye care products. It has also been shown that when tested for antimicrobial activity against *E*. *coli* in the presence and absence of lysozyme in borate buffered saline, that the presence of lysozyme enhanced the effectiveness of PG-3. This effect was more pronounced when the PG-3 was autoclaved and similar patterns were obtained for both the free-acid form and the amide. Accordingly, the protegrins may be used as preservatives in such compositions or as antimicrobials for treatment of eye infections.

It is particularly important that the protegrins retain their activity under physiological conditions including relatively high saline and in the presence of serum. In addition, the protegrins are not cytotoxic with respect to the cells of higher organisms. These properties, described herein below in the Examples, make them particularly suitable for *in vivo* and therapeutic use.

The protegrins of the invention may also be applied to plants or to their environment to prevent viral- and microbial-induced diseases in these plants. Suitable compositions for this use will typically contain a diluent as well as a spreading agent or other ancillary agreements beneficial to the plant or to the environment.

Thus, the protegrins of the invention may be used in any context wherein an antimicrobial and/or antiviral action is required. This use may be an entirely *in vitro* use, or the peptides may be administered to organisms.

In addition, the antimicrobial or antiviral activity may be generated in *situ* by administering an expression system suitable for the production of the protegrins of the invention. Such expression systems can be supplied to plant and animal subjects using known techniques. For example, in animals, pox-based expression vectors can be used to generate the peptides *in situ*. Similarly, plant cells can be transformed with expression vectors and then regenerated into whole plants which are capable of their own production of the peptides.

A particularly useful property of the protegrins is their activity in the presence of serum. Unlike defensins, protegrins are capable of exerting their antimicrobial effects in the presence of serum.

As shown hereinbelow, the protegrins are capable of inactivating endotoxins derived from gram-negative bacteria -- i.e., lipopolysaccharides (LPS) -- in standard assays. Accordingly, the protegrins may be used under any circumstances where inactivation of LPS is desired. One such situation is in the treatment or amelioration of gram-negative sepsis.

The protegrins of the invention, therefore, represent a peculiarly useful class of compounds because of the following properties:
1) they have an antimicrobial effect with respect to a broad spectrum of target microbial systems, including viruses, including retroviruses, bacteria, fungi, yeast and protozoa.
2) Their antimicrobial activity is effective under physiological conditions - i.e., physiological saline and in the presence of serum.
3) They are not toxic to the cells of higher organisms.
4) They can be prepared in nonimmunogenic form thus extending the number of species to which they can be administered.
5) They can be prepared in forms which are resistant to certain proteases suggesting they are antimicrobial even in lysosomes.
6) They can be prepared in forms that resist degradation when autoclaved, thus simplifying their preparation as components of pharmaceuticals.

The following examples are intended to illustrate but not to limit the invention.

### Example 1

### Isolation of PG-1, PG-2 and PG-3

Fresh porcine blood was collected into 15-liter vessels containing 5% EDTA in normal saline, pH 7.4 as an anticoagulant (33 ml/liter blood). The blood cells were allowed to sediment for 90 minutes at room temperature and the leukocyte-rich supernatant was removed and centrifuged at 200 x g for 5.7 minutes. The pellets were pooled and suspended in 0.84% ammonium chloride to lyse erythrocytes and the resulting leukocytes (70-75% PMN, 5-10% eosinophils, 15-25% lymphocytes and monocytes) were washed in normal saline, resuspended in ice-cold 10% acetic acid at 10⁸/ml, homogenized and stirred overnight at 4°C. The preparation was centrifuged at 25,000 x g for 3 hours at 4°C and the supernatant was lyophilized and weighed.

950 mg (dry weight) of lyophilized extract, which contained 520 mg protein by BCA analysis, was stirred overnight at 4°C in 100 ml of 10% acetic acid and then centrifuged at 25,000 x g for 2 hours. The supernate was removed and passed by pressure through a 50 ml stirred ultracentrifugation cell (Amicon, Danvers MA) that contained a YM-5 filter. The ultrafiltrate (24.5 mg protein by BCA) was concentrated to 3 ml by vacuum centrifugation (SpeedVac Concentrator, Savant Instruments, Hicksville, NY), applied to a 2.5 x 117 cm BioGel P10 column (Bio-Rad, Hercules, CA) and eluted at 4°C with 5% acetic acid.

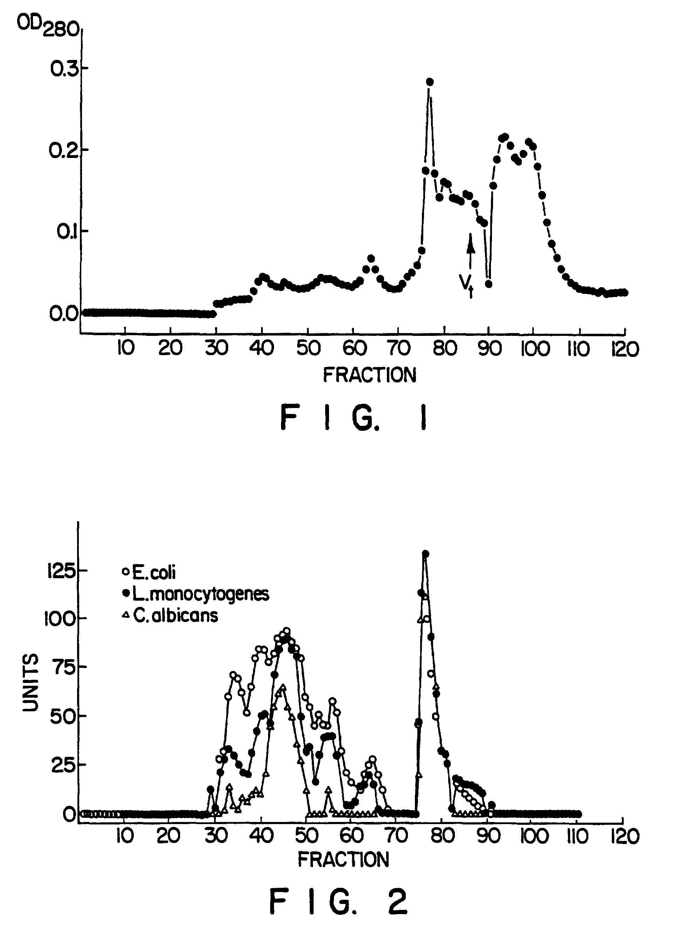

Fractions containing 6.6 ml were obtained. Fractions were assayed by absorption at 280 nm and the elution pattern is shown in Figure 1.

Aliquots (66 *µ*l) of each fraction were dried by vacuum centrifugation and resuspended in 6.6 *µ*l of 0.01% acetic acid. Five *µ*l samples of this concentrate were tested for antimicrobial activity against *E. coli* ML-35, *L. monocytogenes,* strain EGD and *C. albicans,* strain 820, using radiodiffusion and gel overlay techniques as described by Lehrer, R.I. *et al.* J Immunol Meth (1991) 137:167-173. Briefly, the underlay agars used for all organisms had a final pH of 6.5 and contained 9 mM sodium phosphate/1 mM sodium citrate buffer, 1% w/v agarose and 0.30 *µ*g/ml tryptocase soy broth powder (BBL Cockeysville, MD). The units of activity in the radial diffusion assay were measured as described; 10 units correspond to a 1 mm diameter clear zone around the sample well. Activities obtained for the various fractions are shown in Figure 2. Activity was found in a large number of fractions.

The active fractions were further examined by acid-urea PAGE (AU-PAGE) and SDS PAGE. Results of these analyses showed that active antimicrobial peptides of the appropriate molecular weight were present and concentrated in fractions 76-78.

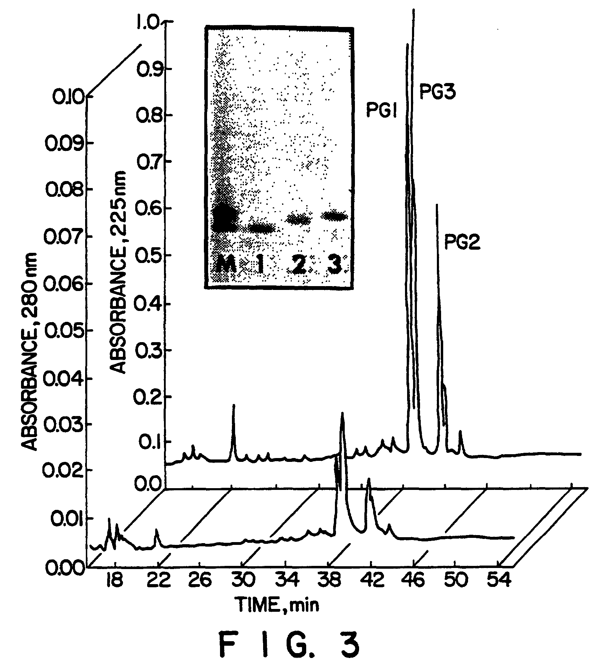

Fractions 76-78 from the Biogel P10 column were then pooled and chromatographed on a 1 x 25 cm Vydac 218 TP1010 column with a gradient (buffer A is 0.1% TFA; buffer B is 0.1% TFA in acetonitrile) the increase in acetonitrile concentration was 1% per minute. The results, assessed in terms of absorbance at 280 nm and at 225 nm are shown in Figure 3. The peaks corresponding the three peptides illustrated herein are labeled in the figure. The figure also contains an inset which shows the results of an acid-urea PAGE gel stained with Comassie Blue that contains a starting mixture composed of the pooled fractions and the individual PG species. These are labeled M, 1, 2 and 3 on the inset. The results clearly show the presence of three distinct proteins.

The isolated proteins were subjected to amino acid analysis using three independent methods, and to Edman degradation, chymotrypsin digestion, and fast atom bombardment mass spectrometric analysis. The peptides, named "protegrins", are shown to have the amino acid sequences as follows: 
PG-1: RGGRLCYCRRRFCVCVGR
PG-2: RGGRLCYCRRRFCICV
PG-3: RGGGLCYCRRRFCVCVGR,
 and are amidated at the C-terminus.

The amidation status of the isolated peptides was established by synthesis of PG-3 both in the free carboxyl and carboxyamidated forms. These synthetic peptides were then compared to isolated PG-3 using AU-PAGE and also using reverse-phase HPLC. In both cases, the native product comigrated with the synthetic amidated form.

The location of the disulfide linkages in the isolated protegrins was also studied using PG-2 as a model. The determination was performed using sequential enzyme digestion (chymotrypsin followed by thermolysin) with direct analysis using LC-ESI-MS on the fragments obtained. The results of these analyses showed that the two intramolecular disulfide bonds were C₆-C₁₅ and C₈-C₁₃. With the location of the disulfides in these positions, the protegrin molecules are likely to exist as anti-parallel β sheets similar to the tachyplesins in overall conformation.

The antimicrobial proteins above are present in much lower concentrations in initial extracts than are the rabbit defensins in corresponding crude extracts where the defensins constitute more than 15% of the total protein in rabbit granulocytes. Using the AU-PAGE analytical method on the various stages of purification, the peptides are only faintly visible in the crude extracts, whereas corresponding crude extracts of rabbit granulocytes clearly show the presence of the defensins. The peptides of the invention become clearly evident only after the ultrafiltration step.

Because the protegrins whose structures are set forth above show sequence homology to the decapeptide region corresponding to residues 1-10 of rabbit defensin NP-3a in the decapeptide region at positions 4-13 of PG-3, the protegrins, and in particular PG-3, may share the property of defensin NP-3a in being capable of competitively antagonizing ACTH-mediated steroid synthesis by adrenocytes. This property, called "corticostasis", may influence the effectiveness of the protegrins as antiinfectious agents when employed *in vivo.*

### Example 2

### Antimicrobial Activity

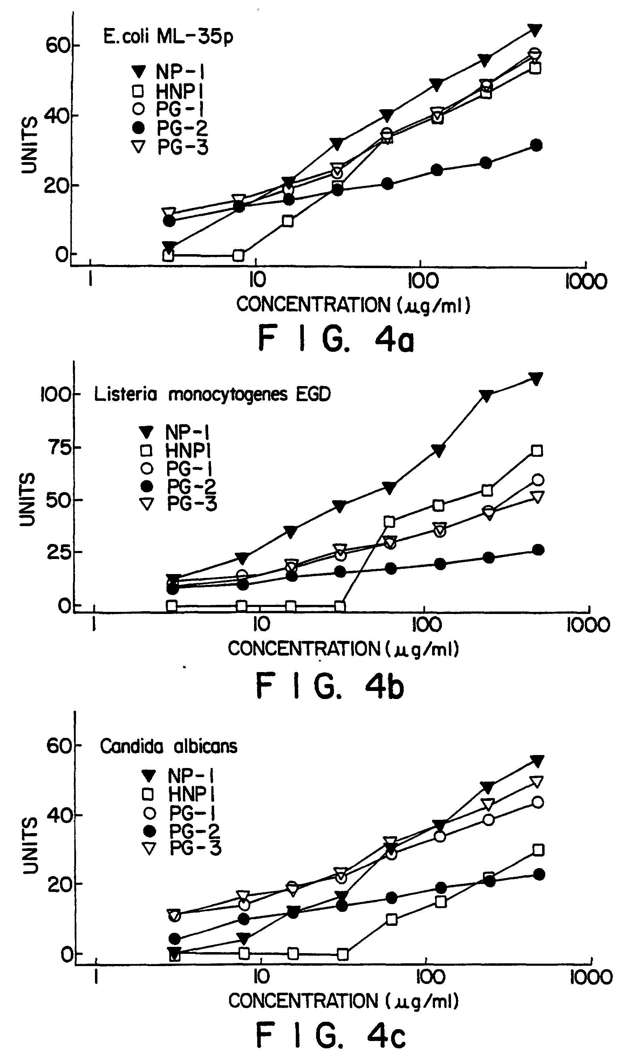

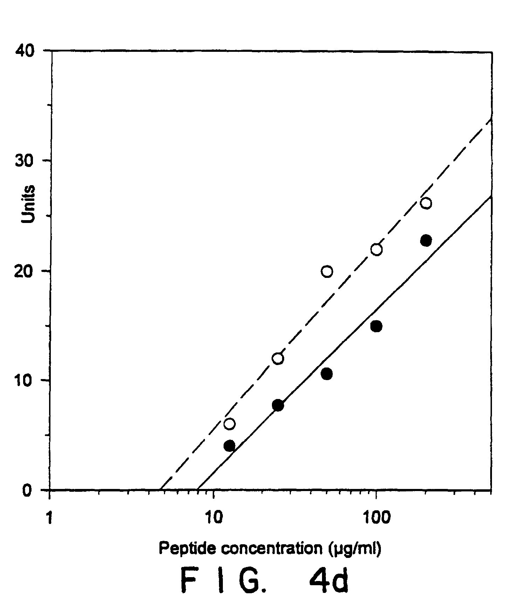

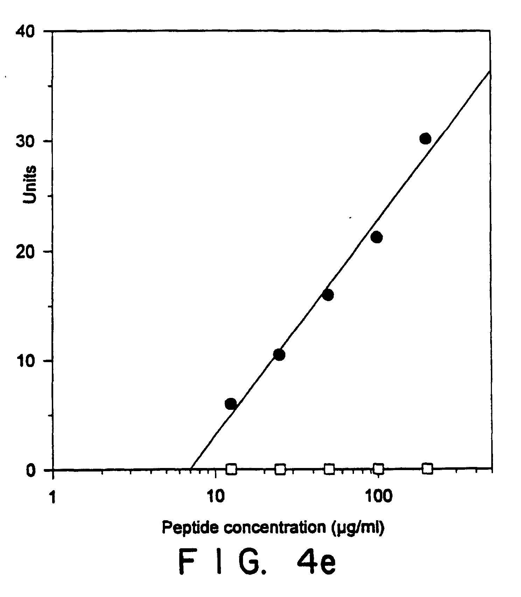

The radial diffusion assay in agarose gels described in Example 1 was also used to test the activity of the purified protegrins. Figures 4a, 4b and 4c show the results against three test organisms in units described as above. The rabbit defensin (NP-1) and the human defensin (HNP-1) were used as controls.

Figure 4a shows that PG-1 and PG-3 are more effective against *E. coli* ML-35P than HNP-1 and only slightly less effective than NP-1. PG-1 and PH-3 were also effective against *Listeria monocytogenes,* strain EGD as shown in Figure 4b. In Figure 4c, PG-1 and PG-3 were also shown effective against *Candida albicans. In* general, these peptides are approximately as effective as rabbit defensin NP-1 on a weight basis and are more effective than HNP-1. In all cases, PG-2 was also effective against the three organisms tested but was not as active as the other two peptides.

In addition to its activity in inhibiting the growth of the above-mentioned organisms, the PG-1 of the invention has been shown directly to inhibit the growth of *Staphylococcus aureus* (see Figure) and *K. pneumoneae* 270 (Figure). HNP-1 used as a control was less effective against *S. aureus* and almost entirely ineffective against *K. pneumoneae.*

The protegrins of the invention have also been tested against various other organisms and show broad spectrum activity. In addition to their effectiveness in inhibiting the growth of or infection by microorganisms associated with STDs as described in Example 9 hereinbelow, the protegrins show strong activity against the following microorganisms in addition to those tested hereinabove: *Pseudomonas aeruginosa, Klebsiella pneumoniae, Salmonella typhimurium, Staphylococcus aureus, Histoplasma capsulatum, Myobacterium avium-intracellulare,* and *Mycobacterium* *tuberculosis*. The protegrins showed only fair activity against *Vibrio vulnificus* and were inactive against *Vibrio cholerae* and *Borrelia burgdorferi.*

### Example 3

### Retention of Activity Under Certain Conditions

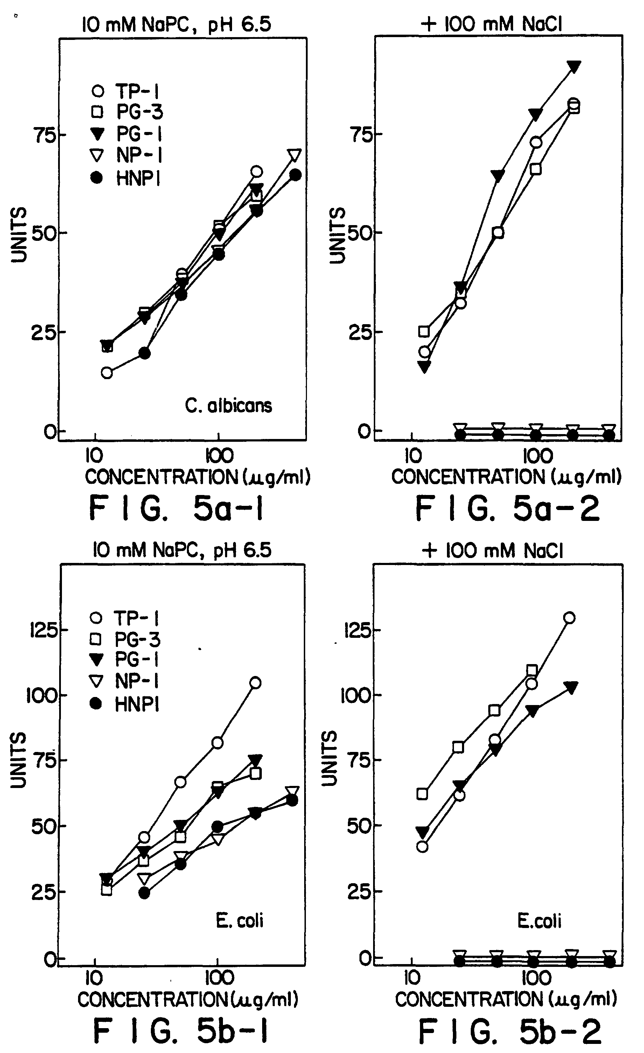

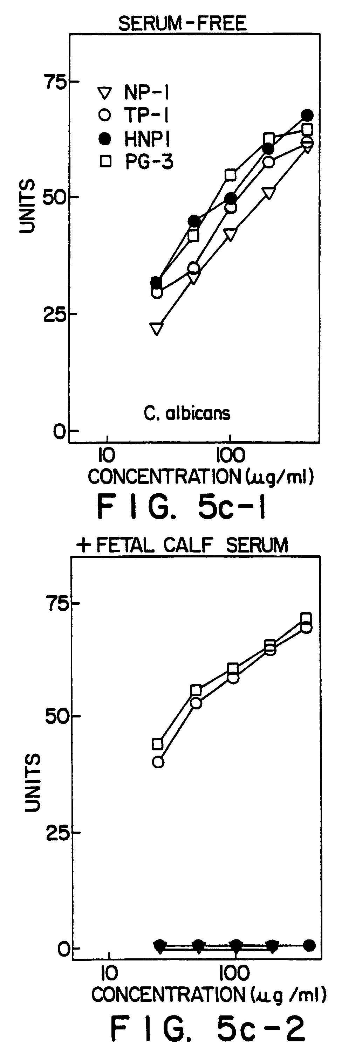

The antimicrobial activity of the invention compounds was tested as set forth above, but under conditions of 100*µ*M NaCl and in the presence of 90% fetal calf serum. Figures 5a and 5b show -+that PG-1 and PG-3 retain their activity with respect to *C*. *albicans* and *E. coli* respectively, even in the presence of 100mM NaCl. Neither NP-1 nor HNP-1 have this property. Figure 5c shows that although NP-1 and NHP-2 lose their ability to inactivate *C. albicans* in 90% fetal calf serum, inactivation by PG-3 is retained.

Accordingly, the protegrins of the invention retain their antimicrobial properties under useful physiological conditions, including isotonic and borate solutions appropriate for use in eye care products.

In addition, synthetic PG-1 was tested with respect to its activity against *E. coli* ML-35 (serum sensitive) in underlayered gels containing only 10 mM sodium phosphate buffer, pH 7.4 and a 1:100 dilution of trypticase soy broth, both in the presence and absence of 2.5% normal human serum, which is below the lytic concentration for this strain of *E*. *coli.* In the presence of serum, the minimal bacteriocidal concentration was reduced from approximately 1.0 *µ*g/ml to about 0.1 *µ*g/ml. This type of effect was not observed either for a linear fragment of cathepsin G or for the defensin HNP-1.

Similarly, using *C. albicans* as a target organism, underlayers were prepared with 10 mM sodium phosphate with and without 10% normal human serum. The minimal fungicidal concentration fell from about 1.3 *µ*g/ml in the absence of serum to 0.14 *µ*g/ml in its presence. The serum itself at this concentration did not effect *C. albicans.*

Thus, not only is the action of the protegrins not inhibited by the presence of serum, it is enhanced thereby. Similar results were obtained using *L. monocytogenes* as the target organism.

The protegrins PG-1 and PG-3 were incubated for 4 hours at pH 2.0 with 0.5 *µ*g/ml pepsin and then neutralized. The residual antimicrobial activity against *C. albicans, E. coli* and *L. monocytogenes* was assessed and found to be fully retained. Similar experiments show that these compounds are not degraded by human leukocyte elastase or by human leukocyte cathepsin G even when exposed to high concentrations of these enzymes and at a pH of 7.0 - 8.0 favorable for proteolytic activity. In addition, synthetic PG-3 amide and synthetic PG-3 acid were autoclaved and tested for antimicrobial activity against *E*. *coli, L. monocyogenese* and *C*. *albicans;* retaining full antimicrobial activity in all cases. It is possible that the stability of these compounds to protease degradation and to autoclaving is enhanced by the presence of disulfide bonds.

### Example 4

### Ability to Bind Endotoxin

The protegrins of the invention were tested for their ability to bind the lipid polysaccharide (LPS) of the gram-negative bacterium *E. coli* strain 0.55B5. The assay was the *Limulus* amebocyte lysate (LAL) test for endotoxins conducted in the presence and absence of the test compounds. The test was conducted using the procedure described in Sigma Technical Bulletin No. 210 as revised in December 1992 and published by Sigma Chemical Company, St. Louis, MO.

The LAL test is based on the ability of LPS to effect gelation in the commercial reagent E-Toxate™ which is prepared from the lysate of circulating amebocytes of the Horseshoe Crab *Limulus polyphemus.* As described in the technical bulletin, when exposed to minute quantities of LPS, the lysate increases in opacity as well as viscosity and may gel depending on the concentration of endotoxin. The technical bulletin goes on to speculate that the mechanism appears analogous to the clotting of mammalian blood and involves the steps of activation of a trypsin-like preclotting enzymes by the LPS in the presence of calcium ion, followed by enzymic modifications of a "coagulogen" by proteolysis to produce a clottable protein. These steps are believed tied to the biologically active or "pyrogenic" portion of the molecule. It has been shown previously that detoxified LPS (or endotoxin) gives a negative LAL test.

The test compounds were used at various concentrations from 0.25 *µ*g-10 *µ*g in a final volume of 0.2 ml and the test mixtures contained LPS at a final concentration of 0.05 endotoxin unit/ml and E-Toxate™ at the same concentration. The test compounds were incubated together with the LPS for 15 minutes before the E-Toxate™ was added to a final volume after E-Toxate™ addition of 0.2 ml. The tubes were then incubated for 30 minutes at 37°C and examined for the formation of a gel.

Both isolated native protegrins (nPGs) and synthetically prepared protegrins (sPGs) were tested. The sPGs were prepared with a carboxyl group at the C-terminus or with an amidated C-terminus. The nPGs are amidated at the C-terminus. Also tested were six different rabbit defensins (NPs) and four native human defensins (HNPs). The results are shown in Table 1.

**Table 1**

| **Peptide** | **10 *µ*g** | **5 *µ*g** | **2.5 *µ*g** | **1.0 *µ*g** | **0.5 *µ*g** | **0.25 *µ*g** |
|---|---|---|---|---|---|---|
| nPG-1 | no gel | no gel | no gel | no gel | + | ++ |
| nPG-2 | no gel | no gel | no gel | no gel | + | ++ |
| nPG-3 | no gel | no gel | trace | ++ | ++ | ++ |
| sPG-3 acid | no gel | no gel | trace | ++ | ++ | ++ |
| sPG-3 amide | no gel | no gel | no gel | + | ++ | ++ |
| NP-1 | not tested | not tested | ++ | ++ | ++ | ++ |
| NP-2 | trace | + | + | ++ | ++ | ++ |
| NP-3a | no gel | no gel | no gel | ++ | ++ | ++ |
| NP-3b | no gel | no gel | + | ++ | ++ | ++ |
| NP-4 | not tested | not tested | + | ++ | ++ | ++ |
| NP-5 | no gel | trace | + | + | ++ | ++ |
| HNP-1 | no gel | + | + | ++ | ++ | ++ |
| HNP-2 | trace | trace | trace | + | + | ++ |
| HNP-3 | no gel | + | + | ++ | ++ | ++ |
| HNP-4 | no gel | trace | trace | ++ | + | ++ |

As seen from the results, all of the protegrins, both synthetic and native, and both in the amidated and nonamidated forms are able to bind sufficiently to LPS to prevent any substantial gel formation at concentrations as low as 2.5 *µ*g/0.2 ml. nPG-1 and nPG-2 are effective at somewhat lower concentrations. The protegrins were substantially more effective than the NP or HNP test compounds; the most effective among these controls was NP-3a, a peptide whose primary sequence most closely resembles that of the protegrins.

In a follow-up experiment, the concentration of LPS was varied from 0.05-0.25 endotoxin units (E.U.) and synthetic PG-3 amide was used as the test compound. The results are shown in Table 2.

**Table 2**

| **Endotoxin Units** | **0.25 E.U.** | **0.10 E.U.** | **0.05 E.U.** |
|---|---|---|---|
| sPG-3 amide (2.5 *µ*g) | no gel | no gel | no gel |
| sPG-3 amide (1.0 *µ*g) | no gel | no gel | no gel |
| sPG-3 amide (0.5 *µ*g) | ++ | ++ | no gel |
| no added protein | ++ | ++ | ++ |

These results show that since inhibition of gelation can be overcome by increasing the concentration of LPS, interaction with LPS is responsible for the lack of gelation, rather than interfering with the gelation enzyme cascade.

### Example 5

### Activity of Linearalized Forms

nPG-1 and nPG-3 were converted to linear form using a reducing agent to convert the disulfide linkages to sulfhydryl groups, which were then stabilized by alkylating with iodoacetamide.

The ability of both cyclic and linearalized PG-1 and PG-3 to inhibit gelation in the standard LAL assay was assessed then as described in Example 4 and the results are shown in Table 3.

**Table 3**

| **Peptide** | **5 *µ*g** | **2.5 *µ*g** | **1.0 *µ*g** | **0.25 *µ*g** | · |
|---|---|---|---|---|---|
| nPG-1 | no gel | no gel | ++ | ++ | ++ |
| cam-nPG-1 | no gel | no gel | ++ | ++ | ++ |
| nPG-3 | no gel | no gel | ++ | ++ | ++ |
| cam-nPG-3 | no gel | no gel | ++ | ++ | ++ |

These results show that the linearalized and cyclic forms of the protegrins are equally capable of inhibiting gelation and binding to endotoxin.

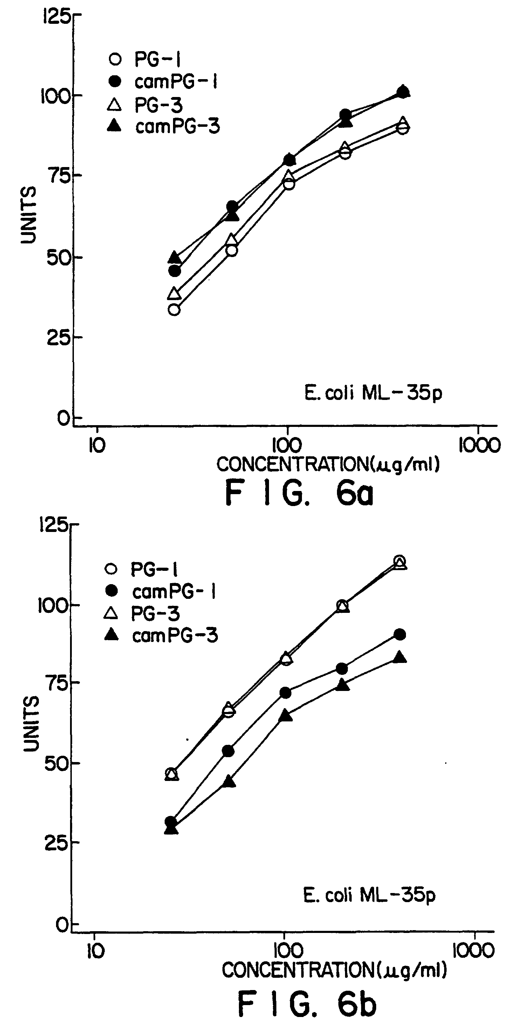

The antimicrobial activity of the linearalized forms was also compared with that of the native protegrins. Both linearalized and cyclic forms of the protegrins tested continue to show antimicrobial activity, although the effectiveness of these peptides as antimicrobials depends on the nature of the target organism and on the test conditions. The antimicrobial activity of native PG-1 and its linearalized form (*cam*-PG-1) and PG-3 and its linearalized form (*cam*-PG-3) were tested according to the procedure set forth in Example 1 as described by Lehrer, R.I. *et al.* J Immunol Meth (1991) 137:167-173. The results are set forth in Figures 6a-6f.

Figures 6a and 6b show the antimicrobial activity of these peptides in the concentration range 20 *µ*g/ml-125 *µ*g/ml with respect to *E*. *coli* ML-35P either in 10 mM phosphate-citrate buffer, pH 6.5 (Figure 6a) or in the presence of this buffer plus 100 mM NaCl (Figure 6b). Both protegrins showed strong antimicrobial activity with respect to this organism; the linear form was slightly more potent in the presence of buffer alone than was the cyclic form; on the other hand, the cyclic form was more potent than the linear form under isotonic conditions.

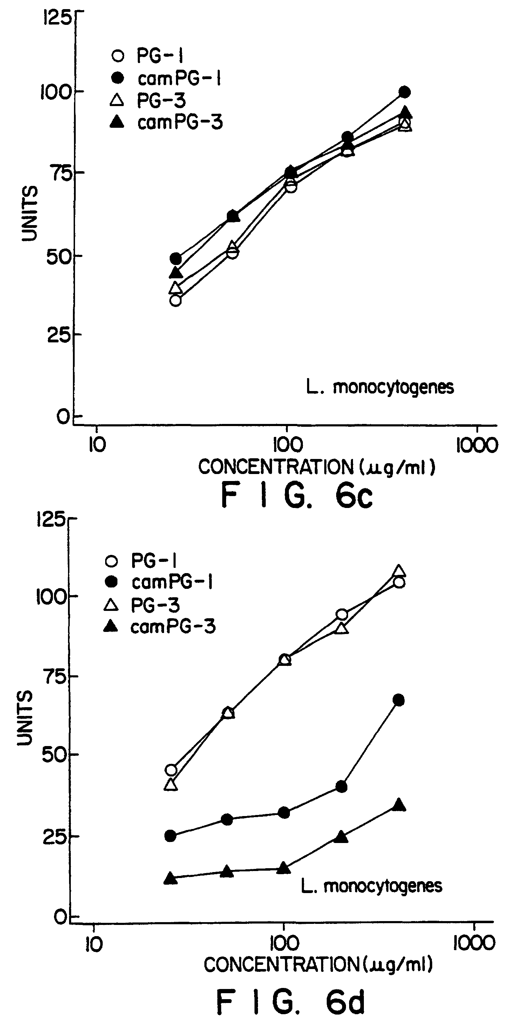

Figures 6c and 6d show the antimicrobial effect with respect to *L. monocytogenes.* In Figure 6c where the above-mentioned buffer alone was used, both cyclic and linearalized forms of the protegrins showed strong antimicrobial activity and both were approximately equally effective over the concentration range tested (20 *µ*g/ml-125 *µ*g/ml).

Figure 6d shows the effect with respect to *L. monocytogenes* in the presence of this buffer plus 100 mM NaCl over the same concentration range. The cyclic form retained strong antimicrobial activity with a slightly greater concentration dependence. Linearalization appeared to lower the activity appreciably although high concentrations were still able to show an antimicrobial effect.

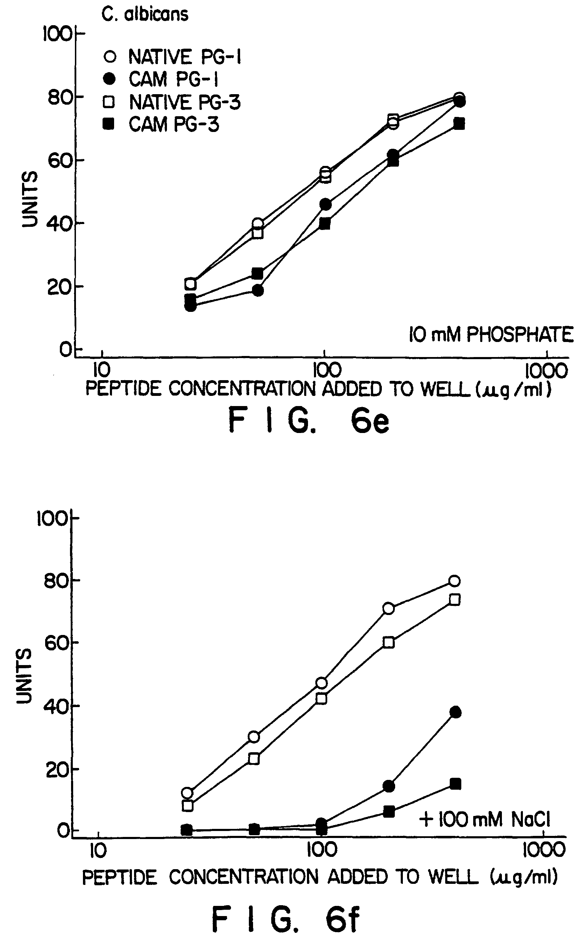

The yeast *C. albicans* was tested with the results shown in Figures 6e and 6f. Figure 6e shows that all forms of these protegrins were antimicrobial in a dose-dependent manner over the above concentration range when tested in the presence of 10 mM phosphate buffer alone, although the linearalized peptides were very slightly less effective. Figure 6f shows the results of the same assay run in the presence of buffer plus 100 mM NaCl. While the cyclized forms retained approximately the same level of antimicrobial effect, the activity of the linearalized forms was greatly diminished so that at concentrations below 100 *µ*g/ml of the protegrin, virtually no antimicrobial effect was seen. However, at higher concentrations of 130 *µ*g/ml, a moderate antimicrobial effect was observed.

Thus, depending on the target microorganism and the conditions used, both the cyclized and linearalized forms of the protegrins have antimicrobial activity.

### Example 6

### Antimicrobial Activity Under Conditions Suitable for Treatment of the Eye

Contact lens solutions are typically formulated with borate buffered physiological saline and may or may not contain EDTA in addition. Protegrins in the form of the synthetic PG-3 amide and synthetic PG acid were tested generally in the assay described in Example 1 wherein all underlay gels contain 25 mM borate buffer, pH 7.4, 1% (v/v) tryptocase soy broth (0.3 *µ*g/ml TSB powder) and 1% agarose. Additions included either 100 mM NaCl, 1 mM EDTA or a combination thereof. Other test compounds used as controls were the defensin NP-1 and lysozyme. Dose response curves were determined.

Table 4 shows the estimated minimal bacteriocidal concentrations in *µ*g/ml of the various test compounds.

**Table 4**

| **ESTIMATED MINIMAL FUNGICIDAL CONCENTRATIONS (*µ*g/ml)** | | | | |
|---|---|---|---|---|
| **Peptide** | **buffer** | **+ EDTA** | **+ NaCl** | **+ EDTA & NaCl** |
| sPG-3 amide | 13.0 | 9.5 | 4.1 | 3.1 |
| sPG-3 acid | 15.0 | 9.5 | 4.6 | 3.7 |
| NP-1 | 35.0 | 45.0 | >200 | >200 |
| lysozyme | 75.0 | 45.0 | >200 | >200 |

Although protegrins are somewhat less active in 25 mM borate buffered saline than in 25 mM phosphate buffer, the antimicrobial activity is enhanced by adding physiological saline and modestly enhanced by 1 mM EDTA, as shown in the table.

A similar test was run with *Candida albicans* as the target organism with the results shown in Table 5, which also shows estimates of minimal fungicidal concentrations.

**Table 5**

| **ESTIMATED MINIMAL FUNGICIDAL CONCENTRATIONS (*µ*g/ml)** | | | |
|---|---|---|---|
| **Peptide** | **25 mM borate buffer** | **borate buffer +120 mM NaCl** | **borate buffer +EDTA & NaCl** |
| nPG-3 | 32.0 | 9.0 | 8.0 |
| sPG-3 amide | 19.0 | 7.7 | 7.0 |
| sPG-3 acid | 19.0 | 9.2 | 9.3 |
| NP-1 | 23.0 | 60.0 | 65.0 |
| HNP-1 | 25.0 | >200 | >200 |

Table 6 shows results of similar experiments conducted with *L. monocytogenes* as the target.

**Table 6**

| **ESTIMATED MINIMAL BACTERICIDAL CONCENTRATIONS (*µ*g/ml)** | | | |
|---|---|---|---|
| **Peptide** | **25 mM borate buffer** | **borate buffer +120 mM NaCl** | **borate buffer +EDTA & NaCl** |
| nPG-3 | 25.0 | 7.0 | 5.7 |
| sPG-3 amide | 21.0 | 5.7 | 5.2 |
| sPG-3 acid | 30.0 | 7.0 | 7.0 |
| NP-1 | 20.0 | 11.0 | 3.8 |
| HNP-1 | 11.0 | >200 | >200 |

The results shown indicate that these compounds are capable of exerting their antimicrobial effects under conditions typically associated with conditions suitable for eye care products.

### Example 7

### Recovery of cDNA Clones and of a New Protegrin-Encoding cDNA cDNA Generation and PCR Amplification.

Total RNA was extracted from the bone marrow cells of a young red Duroc pig with guanidinium thiocyanate.
One *µ*g of total RNA was used to synthesize the first strand cDNA, with 20 pmol Oligo(dT) primer and 200 U Moloney-murine leukemia virus (M-MLV) reverse transcriptase (Clontech Laboratory, Palo Alto, CA) in a total reaction volume of 20 *µ*l. Two PCR primers were prepared. The sense primer (5'-GTCGGAATTCATGGAGACCCAGAG (A or G) GCCAG-3') corresponded to the 5' regions of PG-2 and PR-39 cDNA and contained an EcoRI restriction site. The antisense primer (5'-GTCGTCTAGA (C or G) GTTTCACAAGAATTTATTT-3') was complementary to 3' ends of PG-2 and PR-39 cDNA immediately preceding their poly A tails and contained an XbaI restriction site. PCR was carried out in a 50 *µ*l volume using 1/10 volume of the above pig cDNA as template, 25 pmol primers and 2.5 units of AmpliTaq DNA polymerase (Perkin Elmer-Cetus). The reaction was run for 30 cycles, with 1 min denaturation (94°C) and annealing (60°C) steps and a 2 min extension step (72°C) per cycle.

cDNA Cloning and Sequencing. The amplified cDNA was fractionated by preparative agarose electrophoresis and stained with ethidium bromide.
The main fragment was cut out, digested with EcoR I and Xba I endonucleases (New England Biolabs, Beverly, MA), subcloned into a M13mp18 bacteriophage vector, and transformed into *E*. *coli* XL1-Blue MRF' competent cells (Stratagene, La Jolla, CA). DNA sequencing was performed with a kit (U.S. Biochemical Corp., Cleveland, OH). Nucleotide and protein sequences were analyzed with PC-GENE (Intelligenetics, Palo Alto, CA).

Northern blots. Ten µg of total RNA was denatured in 50% formamide, separated by electrophoresis through 1% agarose gels in 0.62 M formaldehyde, and blotted onto GeneScreen Plus membranes (DuPont, Boston, MA) by capillary transfer. The membrane was baked at 80°C for 2 h, and hybridized with ³²P-labeled probe in rapid hybridization buffer (Amersham, Arlington Height, IL).

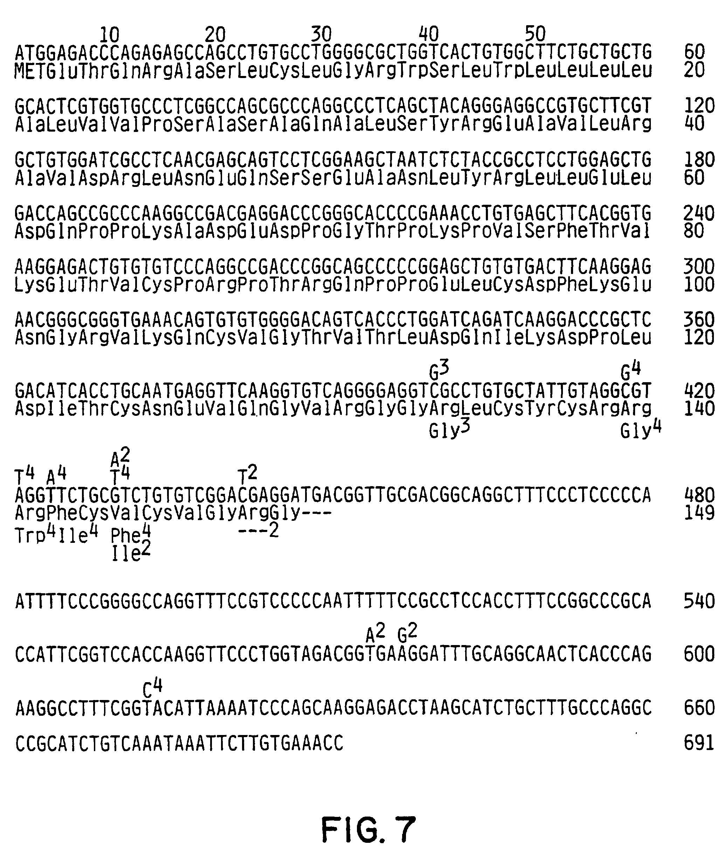

The results of sequencing the various clones encoding the various protegrins is summarized in Figure 7. The cDNA sequences of protegrins PG-1, PG-3 and PG-4 contain 691 bases as had previously been shown for PG-2 by Storici, P. *et al.* Biochem Biophys Res Comm (1993) 196:1363-1368. The cDNAs show an upstream sequence encoding 110 amino acids which appears identical for all protegrins. Additional differences which are quite slight in nature are shown in Figure 7.

The analysis showed the presence of an additional protegrin having an amino acid sequence of Formula (1) wherein A₁₀ is a small amino acid and A₁₁ is a hydrophobic amino acid as distinguished from the previously known protegrins where these residues are basic. The amino acid sequence of PG-4 is therefore RGGRLCYCRGWICFCVGRG, wherein 1, 2, or 3 amino acids at the N-terminus may be deleted.

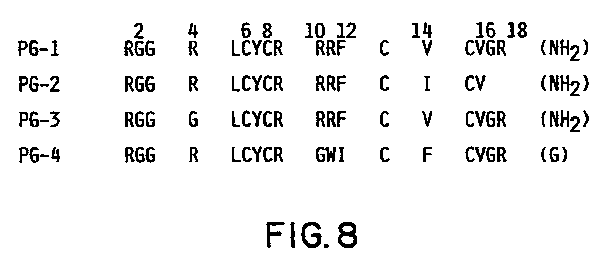

Figure 8 shows a comparison of the amino acid sequences of the four protegrins found so far in porcine leukocytes. There is complete homology in positions 1-3, 5-9, 13 and 15-16.

Additional clones were obtained by amplifying reverse transcribed porcine bone cell RNA using an upstream primer that corresponds to the 5' end of PG-2 and another cathelin-associated peptide, PR39, (Agerbeth B et al., Eur J Biochem (1991) 202:849-854; Storici, P et al., Biochem Biophys Res Com (1993) 186:1058-1065) and downstream primer that matches the region immediately preceding the poly A region. The resulting approximately 0.7 kb PCR product was subcloned into M13mp18 and recombinant plaques were chosen for purification and sequencing. In this manner, the sequences for the precursors of PG-1, PG-3 and PG-4 were recovered. All of these peptides are encoded by a nucleotide sequence which encodes a precursor containing additional amino acid sequence upstream of A₁ of the compound of formula 1 (as shown for PG-4 in Figure 7).

### Example 8

### Preparation of EnantioPG-1

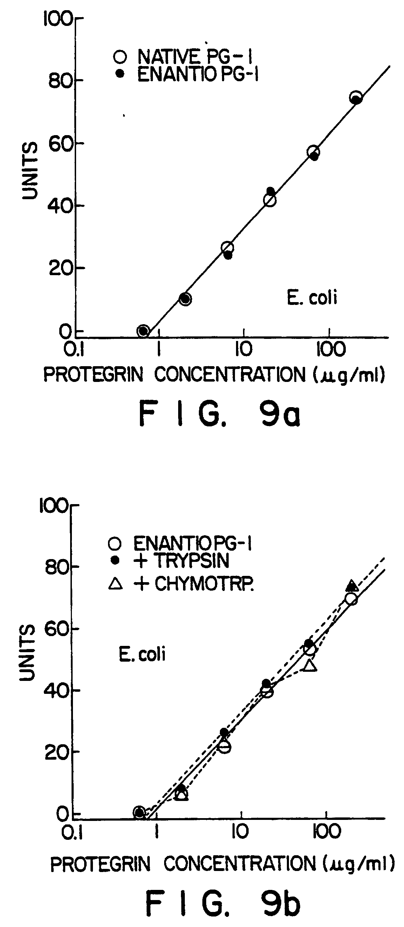

Using standard solid phase techniques, a protegrin having the amino acid sequence of PG-1, but wherein every amino acid is in the D form was prepared. This form of protegrin was tested against *E. coli, L. monocytogenes, C. albicans* and other microbes in the absence and presence of protease and otherwise as described for the radiodiffusion assay in agarose gels set forth in Example 1. The results are shown in Figures 9a-9g.

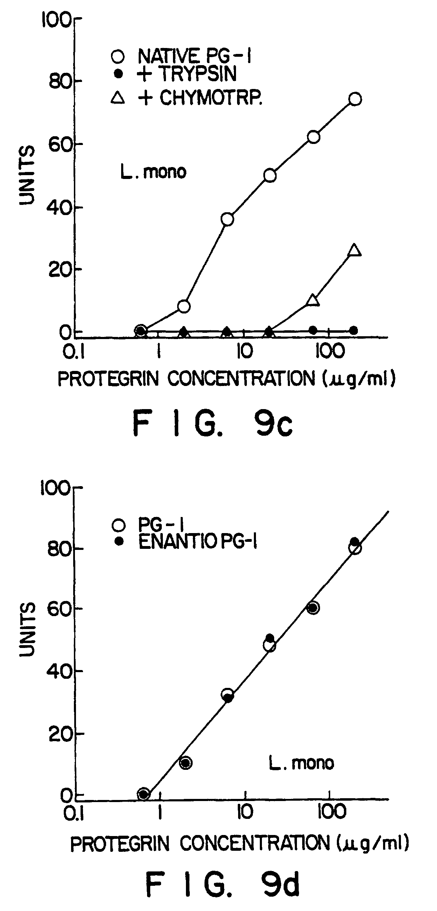

Figure 9a shows that both native PG-1 and *enantio*PG-1 in the absence of protease are equally effective in inhibiting the growth of *E*. *coli*. Figure 9b shows that neither trypsin nor chymotrypsin inhibits the antibacterial effect of *enantio*PG-1. Figure 9c shows that in the presence of these proteolytic enzymes, the ability of native PG-1 to inhibit the growth of *L. monocytogenes* is adversely affected, although, as shown in Figure 9d, in the absence of these proteases PG-1 is comparably active to an *enantio*PG-1.

### Example 9

### Activity of the Protegrins Against STD Pathogens

Table 7 summarizes the activity of the protegrin PG-1 as compared to the defensin HNP-1 against growth of STD pathogens. In these results, "active" means that the peptide was effective at less than 10 *µ*g/ml; moderately active indicates that it was active at 10-25 *µ*g/ml; and slightly active means activity at 25-50 *µ*g/ml. If no effect was obtained at 50-200 *µ*g/ml the compound was considered inactive.

**Table 7**

| Activity against human STD pathogens | Protegrin PG-1 | Defensin HNP-1 |
|---|---|---|
| HIV-1 | Active | Slightly active |
| *Chlamydia trachomatis* | Active | Slightly active |
| *Treponema pallidum* | Active | Inactive |
| *Neisseria gonorrhoeae* | Active | Inactive |
| *Trichomonas vaginalis* | Moderately active | Inactive |
| Herpes simplex type 2 | Moderately active | Slightly active |
| Herpes simplex type 1 | Inactive | Slightly active |
| *Hemophilus ducreyi* | Not tested | Not tested |
| Human papilloma virus | Not tested | Not tested |

### Chlamydia trachomatis

Unlike other bacteria associated with STDs, *Chlamydia* requires an intracellular habitat for metabolic activity and binary fission. The life cycle is as follows: there is an extracellular form which is a metabolically inactive particle somewhat sporelike in its behavior, referred to as an elementary body (EB). The EB attaches to the host cell and is ingested to form an internal vacuolar space often called an "inclusion". The bacterium reorganizes to the delicate reticulate body (RB) which is noninfective but metabolically active and which over a 48-72 hour period undergoes reformation to the EB state. The EBs are then released from the cell. Rather than a peptidoglycan layer, *Chlamydia* contains multiple disulfide linkages in cysteine-rich proteins for protection in the EB stage.

The protegrins of the invention were tested for their antimicrobial activity against *Chlamydia* using the "gold standard" chlamydial culture system for clinical specimens described by Clarke, L.M. in Clinical Microbiology Procedures Handbook II (1992), Isenberg, H.T. Ed. Am. Soc. Microbiol. Washington, D.C.; pp. 8.0.1 to 8.24.3.9. Briefly, McCoy cells (a mouse cell line) in cycloheximide EMEM with 10% fetal bovine serum (FBS) are used as hosts. Prior to chlamydial inoculation, the maintenance medium is aspirated without disruption of the cell layer and the cell layer is maintained on a cover slip in a standard vial. Each vial is then inoculated with 100-300 *µ*L inoculum and centrifuged at 3500 x g for one hour at 20°C. The fluid is then aspirated and 1 ml of EMEM is added. The vials are capped and incubated at 37°C for 48 hours. After 48 hours the medium is again aspirated, coverslips are rinsed twice with PBS and fixed with 300 *µ*L EtOH for 10 minutes. The EtOH is aspirated and the vials are allowed to dry; then one drop PBS plus 30 *µ*L Syva Microtrak monoclonal antibody to the major outer membrane protein of *Chlamydia* is added for staining. After 37°C incubation for 30 minutes, the cells are washed with distilled water and examined for inclusions which are easily recognizable as bright, apple-green-staining cytoplasmic vacuoles. They represent the equivalent of a colony of free-living bacteria on standard bacterial culture media.

In the assays conducted below, *C. trachomatis* serovar L2 (L2/434Bu) described by Kuo, C.C. *et al.* in Nongynococcal Urethritis and Related Infections (1977), Taylor-Robinson, D. *et al.* Ed. Am. Soc. Microbiol. Washington, D.C., pp. 322-326 was used. The seed is prepared from a sonicated culture in L929 mouse fibroblast cells, and partially purified by centrifugation. Since host protein is still present in the seed aliquots, each seed batch is titered at the time of preparation with serial tenfold dilutions to 2 x 10⁻⁹. The seed containing 9.2 x 10⁶ IFU/ml is thawed quickly at 37°C and diluted to 10⁻² with sucrose/phosphate salts/glycine to produce IFU of about 200 after room temperature preincubation and to dilute background eukaryotic protein.

In the initial assays, the peptides to be tested were prepared as stock solutions in 0.01% glacial acetic acid. 100 *µ*L of the diluted chlamydial seed was aliquoted into 1.5 ml eppendorf tubes and 200 *µ*L of the antibiotic peptide was added per tube. Aliquots of the peptide stock (and controls) were incubated with the seed at room temperature for one hour, two hours and four hours. About 10 minutes before the end of each incubation period, maintenance media were aspirated from the McCoy vials in preparation for standard inoculation and culture. Culture was then performed in the presence and absence of the peptides; in some cases, the peptides were added to final concentration in the culture media in addition to the preculture incubation. The test was evaluated microscopically.

The results using 50 *µ*g of protegrin per addition were dramatic. In control cultures, where no peptides were added, 222-460 inclusions were counted. In all protocols where protegrin was added either before the *Chlamydia* seed was added to the cells or both before and after, no inclusions were found. Similar results were obtained with 20 *µ*g additions of tachyplesin. The defensins MP-1 and HNP-1 had lesser protective effects. In summary, the protegrins tested show antimicrobial against *Chlamydia.*

In the next series of experiments, various concentrations of protegrin (1 *µ*g, 12.5 *µ*g, 25 *µ*g and 50 *µ*g) were used in the two-hour preincubation. Concentrations as low as 12.5 *µ*g lowered the number of inclusions to zero. Even at a concentration of 1 *µ*g/ml, the number of inclusions was lowered dramatically from about 110 to about 30.

In the next set of experiments, the effect of the presence of serum was tested. The *Chlamydia* seed was preincubated for two hours with and without 10% FBS and also with or without protegrin at 25 *µ*g. Protegrin was highly effective both with and without serum, whereas human defensin HNP-2, used as a control, was reasonably effective in the absence of serum but only marginally effective in its presence.

The experiments were repeated but adding 25 *µ*g of protegrin one after the start of the chlamydial culture, i.e., after centrifugation and final medium mix and one hour into the beginning of the 48-hour culture period. Protegrin reduced the number of inclusions by approximately 57% from untreated controls although HNP-2 was completely ineffective. Finally, the protegrin (at 25 *µ*g) was added to the chlamydial seed and the mix then immediately cultured. In this case, without preincubation and without the one-hour post-infection gap, protegrin was minimally effective without or without serum.

The effect of serum is particularly important since for a topical agent to be effective in combatting *Chlamydia* infection, it must act in the presence of serum.

In addition, there are several mouse-based models for *Chlamydia* infection which can be used to assess the efficacy of the protegrins. These include those described by Patton, D.L. *et al.* in Chlamydial Infections (1990) Bowie, W.R. *et al.* Eds. Cambridge University Press NY pp. 223-231; Swenson, C.E. *et al.* J. Infect. Dis. (1983) pp. 1101-1107, and Barron, A.L. *et al.* J. Infect. Dis. (1981) 143:63-66.

### Neisseria gonorrhoeae

In more detail, the ability of the protegrins to inhibit *N. gonorrhoeae* was tested by a modification of the method of Miyasaki et al., Antimicrob Agent Chemother (1993) 37:2710-2715. Nonpiliated transparent variants of strains FA 19 and F 62 were propagated on GCB agar plates containing glucose and iron supplements overnight at 37°C under 3.8% V/V CO2. These strains were chosen for their adaptability to the assay.

The overnight growth is removed from the agar plate and suspended in GCB broth containing supplements and sodium bicarbonate and grown with shaking at 37°C to mid log phase. The culture is diluted 1:100 in GCB broth to give about 10⁶ CFU/ml and serial dilutions were plated onto GCB agar.

The peptides are dissolved in 0.01% v/v acetic acid to give a 1 mg/ml stock solution and serially diluted. Ten *µ*l of each dilution is added to a sterile polystyrene tube containing 90 *µ*l of diluted bacteria and the tubes are shaken at 37°C for 45 minutes. The contents are serially diluted 1:10 and plated on to GCB agar plates which are incubated in a CO₂ incubator. CFU are counted after 24 hours and the log bactericidal activity calculated.

Native PG-1, synthetic PG-1, synthetic PG-3 amide and synthetic PG-3 without amidation all gave over a 5 log reduction in CFU per ml in this assay. Native PG-2 (containing 16 amino acids) gave a 2.6 fold reduction.

In addition *enantio*PG-1, the unidisulfide PG-1 (C₆-C₁₅), and unisulfide PG-1 (C₈-C₁₃) gave over a 5-fold log reduction in CFU/ml in this assay.

### Treponema pallidum

Bacteriocidal activity against this organism, which is the etiologic agent of syphilis, was also tested. Peptides were evaluated at a series of concentrations of 1.758 *µ*g to 56.25 *µ*g in 90 *µ*l of unheated normal rabbit serum. The serum served as a nutrient for the spirochetes to allow their survival during incubation as well as providing a source of complement. Ten *µ*l of a suspension of *T. pallidum* containing about 5 x 10⁷/*µ*l organisms was added to each tube and the mixtures with the appropriate peptides were incubated at 34°C under 95% N₂ and 5% CO₂. At time zero, just prior to incubation, 4 hours and 16 hours, 25 randomly selected organisms were examined for the presence or absence of motility. The 50% immobilizing end point (IE₅₀) was calculated to indicate the concentration needed to immobilize 50% of the spirochetes. In the presence of PG-1, the IE₅₀ at 0 and 4 hours was 2.717 *µ*g and < 1.758 *µ*g, respectively. Tachyplesin IE₅₀'s were 5.231 *µ*g and 2.539 *µ*g for 0 and 4 hours. This was in contrast to HNP and NP preparations which showed little immobilizing ability.

### Herpes Simplex Virus

Using viral stocks prepared in VERO cells, grown in minimal essential medium (MEM) with 2% fetal calf serum, the effect of various peptides on HSV 1 MacIntyre strain, a pool of ten clinical HSV 1 isolates, HSV-2G, and a pool of ten clinical HSV 2 isolates, all sensitive to 3 *µ*M acyclovir were tested. Two fibroblast cell lines, human W138 and equine CCL57, were used as targets and tests were done by direct viral neutralization and delayed peptide addition.

In the direct neutralization format, the virus was preincubated with the peptides for 90 min before it was added to the tissue culture monolayers. In the delayed peptide addition format, the virus was added and allowed 50 min to adsorb to the target cells, then the monolayers were washed and peptides were added for 90 min. Finally, the monolayer was washed to remove the peptide and the cells were fed with peptide-free MEM and cultured until the untreated infected monolayers exhibited 4+ cytopathic effect (CPE) (about 60 hours).

Antiviral activity was seen in both formats, but was more pronounced with the delayed peptide addition mode. In experiments performed with W138 and CCL57 cells in the direct neutralization format, PG-1 completely prevented HSV-2G from causing CPE at concentrations of 50 *µ*g/ml and 25 *µ*g/ml, but these concentrations afforded no protection against HSV-1, which produced 4+ CPE.

In the delayed peptide addition format, PG-1 completely prevented CPE by HSV-2G at 35 *µ*g/ml and 50 *µ*g/ml and it also fully protected against the clinical HSV-2 pool at both concentrations.

Thus, PG-1 protected human and animal cells from infection by laboratory and clinical strains of HSV-2, even when the peptides were added as late as 60 min after the virus had ben introduced into the cell culture.

### Trichomonas vaginallis

*Trichomonas vaginallis* strain Cl (ATCC 30001) was grown as described by Gorrell, T.E. et al, Carlsberg Res Comm (1984) 49:259-268. In experiments performed in RPMI + 1% heat-activated fetal calf serum, within a few minutes after exposure to 50 *µ*g/ml PG-1, *T. vaginallis* (heretofore vigorously motile) became stationary. Soon thereafter, the organisms became permeable to trypan blue, and, over the ensuing 15-30 minutes, lysed. As expected, such organisms failed to grow when introduced into their customary growth medium (Diamond's medium). Organisms exposed to 25 *µ*g/ml of PG-3 retained their motility.

Initial studies with two highly metronidazole-resistant clinical isolates of *T.* *vaginallis,* strains MR and TV showed both were susceptible to PG-1, including the C₈-C₁₃ and C₆-C₁₅ unidisulfides and *enantio*PG-1 at concentrations of 100 and 50 *µ*g/ml.

### Example 10

### Antiretroviral Activity

Both synthetic and native PG-1 and native PG-2 were tested for antiviral activity against strains of HIV using the method described in Miles, S.A. et al., Blood (1991) 78:3200-3208. Briefly, the mononuclear cell fraction is recovered from normal donor leukopacs from the American Red Cross using a Ficoll-hypaque density gradient. The mononuclear cells are resuspended at 1 x 10⁶ cells per ml in RPMI 1640 medium with 20% fetal bovine serum, 1% penn/strep with fungizone and 0.5% PHA and incubated 24 hours at 37°C in 5% CO₂. The cells are centrifuged, washed and then expanded for 24 hours in growth medium.

Non-laboratory adapted, cloned HIV_{JR-CSF} and HIV_{JR-FL} were electroporated into the human peripheral blood mononuclear cells prepared as described above. Titers were determined and in general, multiplicities of infection (MOI) of about 4,000 infectuous units per cell are used (which corresponds to 25-40 picograms per ml HIV p24 antigen in the supernatant).

In the assay, the HIV stocks prepared as above were diluted to the correct MOI and the PBM are added to 24 well plates at a concentration of 2 x 10⁶ per ml. One µl total volume is added to each well. The peptide to be tested is added in growth medium to achieve the final desired concentration. Then the appropriate number of MOI are added. To assay viral growth, 200 µl of supernatant is removed on days 3 and 7 and the concentration of p24 antigen is determined using a commercial assay (Coulter Immunology, Hialeah, Florida). Controls include dupulicate wells containing cells alone, cells plus peptide at 5 *µ*g/ml cells with virus but not peptide and cells with virus in the presence of AZT at 10⁻⁵ M - 10⁻⁸ M.

Using this assay, it was demonstrated that both natural and synthetic PG-1 completely inhibit HIV infection at concentrations between 1-5 *µ*g/ml; IC₉₀ was < 5 *µ*g/ml. The time of addition of peptide was then varied. Cells pretreated for 2 hours prior to addition of virus, at the time of addition of virus, or 2 hours after infection showed antiviral activity for the peptide. However, if PG-1 was added 24 hours after infection, there was no antiviral activity.

Further, PG-2 shows similar activity but at a level approximately 5-fold less. Alternative antibiotics such as human defensins and rabbit defensins lacked potent activity in this assay. The results were similar for both HIV_{JR-CSF} and HIV_{JR-FL} which are non-laboratory adapted isolates (Koyanagi, Y.S. et al, Science (1987) 236:819-822).

The protegrins show similar activity with respect to other retroviruses.

## Claims

1. A compound of the formula
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1)
and the N-terminal acylated and/or C-terminal amidated or esterified forms thereof, which is either in the optionally -SH stabilized linear or in a cystine-bridged form
wherein each of A₁ and A₉ is independently a basic amino acid;
each of A₂ and A₃ is independently a small amino acid;
each of A₅, A₇, A₁₂, A₁₄ and A₁₆ is independently a hydrophobic amino acid;
each of A₄ and A₁₀ is independently a basic or a small amino acid;
A₁₁ is a basic or a hydrophobic amino acid;
A₁₇ is not present or, if present, is a small amino acid;
A₁₈ is not present or, if present, is a basic amino acid, or a
modified form of formula (1) and the N-terminal acylated and/or C-terminal amidated or esterified forms thereof wherein each of 1-4 cysteines is independently replaced by a hydrophobic amino acid or a small amino acid;
with the proviso that if said compound is of the formula 
RGGRLCYCRRRFCVCVGR,
RGGRLCYCRRRFCICV,
RGGGLCYCRRRFCVCVGR, or
RGGRLCYCRGWICFCVGR
 in the amidated and di-cystine-bridged form, said compound is purified and isolated.

2. The compound of claim 1 which contains two cystine bridges;
and/or wherein the C-terminal carboxyl is of the formula selected from the group consisting of COOH or the salts thereof; COOR, CONH₂, CONHR, and CONR₂ wherein each R is independently hydrocarbyl (1-6C) ;
and/or wherein the amino group at the N-terminus is of the formula NH₂ or NHCOR wherein R is hydrocarbyl (1-6C) ;
and/or wherein each of A₁ and A₉ is independently selected from the group consisting of R, K and Har;
and/or wherein each of A₂ and A₃ is selected independently from the group consisting of G, A, S and T; and/or wherein A₄ is R or G;
and/or wherein each of A₅, A₁₄ and A₁₆ is independently selected from the group consisting of I, V, NLe, L and F;
and/or wherein each of A₇ and A₁₂ is independently selected from the group consisting of I, V, L, W, Y and F;
and/or wherein each of A₁₀ and A₁₁ is independently R, G or W.

3. The compound of claim 1 which contains one cystine bridge;
and/or wherein the C-terminal carboxyl is of the formula selected from the group consisting of COOH or the salts thereof; COOR, CONH₂, CONHR, and CONR₂ wherein each R is independently hydrocarbyl (1-6C) ;
and/or wherein the amino group at the N-terminus is of the formula NH₂ or NHCOR wherein R is hydrocarbyl (1-6C) ;
and/or wherein each of A₁ and A₉ is independently selected from the group consisting of R, K and Har;
and/or wherein each of A₂ and A₃ is selected independently from the group consisting of G, A, S and
T; and/or wherein A₄ is R or G;
and/or wherein each of A₅, A₁₄ and A₁₆ is independently selected from the group consisting of I, V, NLe, L and F;
and/or wherein each of A₇ and A₁₂ is independently selected from the group consisting of I, V, L, W, Y and F;
and/or wherein each of A₁₀ and A₁₁ is independently R, G or W.

4. The compound of claim 1 which is in the linear form;
and/or wherein the C-terminal carboxyl is of the formula selected from the group consisting of COOH or the salts thereof; COOR, CONH₂, CONHR, and CONR₂ wherein each R is independently hydrocarbyl(1-6C);
and/or wherein the amino group at the
N-terminus is of the formula NH₂ or NHCOR wherein R is hydrocarbyl (1-6C) ;
and/or wherein each of A₁ and A₉ is independently selected from the group consisting of R, K and Har;
and/or wherein each of A₂ and A₃ is selected independently from the group consisting of G, A, S and
T; and/or wherein A₄ is R or G;
and/or wherein each of A₅, A₁₄ and A₁₆ is independently selected from the group consisting of I, V, NLe, L and F;
and/or wherein each of A₇ and A₁₂ is independently selected from the group consisting of I, V, L, W, Y and F;
and/or wherein each of A₁₀ and A₁₁ is independently R, G or W.

5. The compound of claim 1 which is in said modified form;
and/or wherein the C-terminal carboxyl is of the formula selected from the group consisting of COOH or the salts thereof; COOR, CONH₂, CONHR, and CONR₂ wherein each R is independently hydrocarbyl (1-6C) ;
and/or wherein the amino group at the N-terminus is of the formula NH₂ or NHCOR wherein R is hydrocarbyl (1-6C) ;
and/or wherein each of A₁ and A₉ is independently selected from the group consisting of R, K and Har;
and/or wherein each of A₂ and A₃ is selected independently from the group consisting of G, A, S and T;
and/or wherein A₄ is R or G;
and/or wherein each of A₅, A₁₄ and A₁₆ is independently selected from the group consisting of I, V, NLe, L and F;
and/or wherein each of A₇ and A₁₂ is independently selected from the group consisting of I, V, L, W, Y and F;
and/or wherein each of A₁₀ and A₁₁ is independently R, G or W.

6. The compound of claim 2 wherein said cystine bridges link C₆-C₁₅ and C₈-C₁₃.

7. The compound of claim 3 wherein said cystine bridges link C₆-C₁₅ or C₈-C₁₃.

8. The compound of claim 5 wherein each of said 1-4 cysteines is independently replaced by G, A, S, T, I, V, or L.

9. The compound of claim 1 which is selected from the group consisting of PG-1: RGGRLCYCRRRFCVCVGR 
PG-2: RGGRLCYCRRRFCICV
PG-3: RGGGLCYCRRRFCVCVGR
PG-4: RGGRLCYCRGWICFCVGR
 and the amidated forms thereof either in linear or cystine-bridged form.

10. The compound of any of claims 1-9 wherein all amino acids are in the D-configuration.

11. A purified and isolated compound of the formula:
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1a)
and the N-terminal acylated, C-terminal amidated or esterified forms thereof in either the linearalized or the cystine-bridged form
wherein each Aₙ is independently an amino acid at the designated position and wherein said cystine-bridged compound is isolatable from the leukocytes of an animal subject by a process which comprises subjecting the ultrafiltrate of a lysate of said leukocytes to chromatographic separation techniques to obtain a fraction containing compounds of Formula (1a) and recovering said compounds of Formula (1a).

12. A peptide in purified and isolated form having antimicrobial and/or antiviral activity which is encoded by a DNA which hybridizes under stringent conditions to the DNA of porcine leukocytes which encodes PG-1, PG-2, PG-3 or PG-4.

13. A purified and isolated DNA which hybridizes under stringent conditions to the DNA of porcine leukocytes which encodes PG-1, PG-2, PG-3 or PG-4.

14. A recombinant expression system for production of a peptide having the amino acid sequence of the compound of any of claims 1-9 which expression system comprises a nucleotide sequence encoding said peptide operably linked to control sequences for effecting expression.

15. A recombinant host cell modified to contain the expression system of claim 14.

16. A method to produce an antimicrobial or antiviral peptide or intermediate peptide therefor which method comprises culturing the modified host cells of claim 15 under conditions wherein said peptide is produced; and
recovering the peptide from the culture.

17. The method of claim 16 which further comprises effecting cystine linkages of said peptide and/or modifying the N-terminus and/or C-terminus of said peptide.

18. A pharmaceutical composition for antimicrobial or antiviral use which comprises the compound of any of claims 1-12 in admixture with at least one pharmaceutically acceptable excipient.

19. A composition for application to plants or plant environments for conferring resistance to microbial or viral infection in plants which comprises the compound of claims 1-12 in admixture with at least one environmentally acceptable diluent.

20. A method to prevent the growth of a virus or microbe, which method comprises contacting a plant or plant environment with an amount of the compound of any of claims 1-12 effective to prevent said growth.

21. The compound of any of claims 1-12 for use in a method of preventing the growth of a virus or microbe in a human or animal.

22. The compound of claim 21 for use as an eye care product.

23. The compound of any of claims 1-12 for use in treating a sexually transmitted disease.

24. The compound of any of claims 1-12 for use in a method to inactivate the endotoxin of gram-negative bacteria in a human or animal.

25. The compound of any of claims 1-12 for use in treating septic shock.

26. Antibodies specifically reactive with the compound of any of claims 1-12.

27. Use of the compound of any of claims 1-12 for the manufacture of a medicament for preventing the growth of a virus or microbe in a human or animal.

## Patentansprüche

1. Eine Verbindung der Formel
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1)
sowie deren N-terminal acylierten und/oder C-terminal amidierten oder veresterten Formen, welche entweder in der gegebenenfalls -SH-stabilisierten linearen oder in einer cystinverbrückten Form vorliegt,
worin jeweils A₁ und A₉ unabhängig voneinander eine basische Aminosäure sind;
jeweils A₂ und A₃ unabhängig voneinander eine kleine Aminosäure sind;
jeweils A₅, A₇, A₁₂, A₁₄ und A₁₆ unabhängig voneinander eine hydrophobe Aminosäure sind;
jeweils A₄ und A₁₀ unabhängig voneinander eine basische oder eine kleine Aminosäure sind;
A₁₁ eine basische oder eine hydrophobe Aminosäure ist;
A₁₇ nicht vorliegt oder, wenn es vorliegt, eine kleine Aminosäure ist;
A₁₈ nicht vorliegt oder, wenn es vorliegt, eine basische Aminosäure ist, oder eine
modifizierte Form der Formel (1) sowie deren N-terminal acylierten und/oder C-terminal amidierten oder veresterten Formen, worin jedes der 1 - 4 Cysteine unabhängig voneinander durch eine hydrophobe Aminosäure oder eine kleine Aminosäure ersetzt ist;
unter der Voraussetzung, dass, wenn die Verbindung die Formel 
RGGRLCYCRRRFCVCVGR,
RGGRLCYCRRRFCICV,
RGGGLCYCRRRFCVCVGR, oder
RGGRLCYCRGWICFCVGR
in der amidierten und di-cystinverbrückten Form aufweist, diese Verbindung gereinigt und isoliert ist.

2. Verbindung nach Anspruch 1, welche zwei Cystinbrücken enthält;
und/oder worin die C-terminale Carboxylgruppe eine Formel aufweist, die ausgewählt ist aus der Gruppe, bestehend aus COOH oder deren Salzen; COOR, CONH₂, CONHR und CONR₂, worin jedes R unabhängig voneinander eine Hydrocarbylgruppe (1-6C) ist;
und/oder worin die Aminogruppe an dem N-Terminus die Formel NH₂ oder NHCOR aufweist, worin R eine Hydrocarbylgruppe (1-6C) ist;
und/oder worin jeweils A₁ und A₉ unabhängig voneinander ausgewählt sind aus der Gruppe, bestehend aus R, K und Har;
und/oder worin jeweils A₂ und A₃ unabhängig voneinander ausgewählt sind aus der Gruppe, bestehend aus G, A, S und T;
und/oder worin A₄ R oder G ist;
und/oder worin jeweils A₅, A₁₄ und A₁₆. unabhängig voneinander ausgewählt sind aus der Gruppe, bestehend aus I, V, NLe, L und F;
und/oder worin jeweils A₇ und A₁₂ unabhängig voneinander ausgewählt sind aus der Gruppe, bestehend aus I, V, L, W, Y und F;
und/oder worin jeweils A₁₀ und A₁₁ unabhängig voneinander R, G oder W sind.

3. Die Verbindung nach Anspruch 1, welche eine Cystinbrücke enthält;
und/oder worin die C-terminale Carboxylgruppe eine Formel aufweist, die ausgewählt ist aus der Gruppe, bestehend aus COOH oder deren Salzen; COOR, CONH₂, CONHR und CONR₂, worin jedes R unabhängig voneinander eine Hydrocarbylgruppe (1-6C) ist;
und/oder worin die Aminogruppe an dem N-Terminus die Formel NH₂ oder NHCOR aufweist, worin R eine Hydrocarbylgruppe (1-6C) ist;
und/oder worin jeweils A₁ und A₉ unabhängig voneinander ausgewählt sind aus der Gruppe, bestehend aus R, K und Har;
und/oder worin jeweils A₂ und A₃ unabhängig voneinander ausgewählt sind aus der Gruppe, bestehend aus G, A, S und T;
und/oder worin A₄ R oder G ist;
und/oder worin jeweils A₅, A₁₄ und A₁₆ unabhängig voneinander ausgewählt sind aus der Gruppe, bestehend aus I, V, NLe, L und F;
und/oder worin jeweils A₇ und A₁₂ unabhängig voneinander ausgewählt sind aus der Gruppe, bestehend aus I, V, L, W, Y und F;
und/oder worin jeweils A₁₀ und A₁₁ unabhängig voneinander R, G oder W sind.

4. Die Verbindung nach Anspruch 1, welche in der linearen Form vorliegt;
und/oder worin die C-terminale Carboxylgruppe eine Formel aufweist, die ausgewählt ist aus der Gruppe, bestehend aus COOH oder deren Salzen; COOR, CONH₂, CONHR und CONR₂, worin jedes R unabhängig voneinander eine Hydrocarbylgruppe (1-6C) ist;
und/oder worin die Aminogruppe an dem N-Terminus die Formel NH₂ oder NHCOR aufweist, worin R eine Hydrocarbylgruppe (1-6C) ist;
und/oder worin jeweils A₁ und A₉ unabhängig voneinander ausgewählt sind aus der Gruppe, bestehend aus R, K und Har;
und/oder worin jeweils A₂ und A₃ unabhängig voneinander ausgewählt sind aus der Gruppe, bestehend aus G, A, S und T;
und/oder worin A₄ R oder G ist;
und/oder worin jeweils A₅, A₁₄ und A₁₆ unabhängig voneinander ausgewählt sind aus der Gruppe, bestehend aus I, V, NLe, L und F;
und/oder worin jeweils A₇ und A₁₂ unabhängig voneinander ausgewählt sind aus der Gruppe, bestehend aus I, V, L, W, Y und F;
und/oder worin jeweils A₁₀ und A₁₁ unabhängig voneinander R, G oder W sind.

5. Die Verbindung nach Anspruch 1, welche in der genannten modifizierten Form vorliegt;
und/oder worin die C-terminale Carboxylgruppe eine Formel aufweist, die ausgewählt ist aus der Gruppe, bestehend aus COOH oder deren Salzen; COOR, CONH₂, CONHR und CONR₂, worin jedes R unabhängig voneinander eine Hydrocarbylgruppe (1-6C) ist;
und/oder worin die Aminogruppe an dem N-Terminus die Formel NH₂ oder NHCOR aufweist, worin R eine Hydrocarbylgruppe (1-6C) ist;
und/oder worin jeweils A₁ und A₉ unabhängig voneinander ausgewählt sind aus der Gruppe, bestehend aus R, K und Har;
und/oder worin jeweils A₂ und A₃ unabhängig voneinander ausgewählt sind aus der Gruppe, bestehend aus G, A, S und T;
und/oder worin A₄ R oder G ist;
und/oder worin jeweils A₅, A₁₄ und A₁₆ unabhängig voneinander ausgewählt sind aus der Gruppe, bestehend aus I, V, NLe, L und F;
und/oder worin jeweils A₇ und A₁₂ unabhängig voneinander ausgewählt sind aus der Gruppe, bestehend aus I, V, L, W, Y und F;
und/oder worin jeweils A₁₀ und A₁₁ unabhängig voneinander R, G oder W sind.

6. Die Verbindung nach Anspruch 2, worin die Cystinbrücken C₆ mit C₁₅ und C₈ mit C₁₃ verknüpfen.

7. Die Verbindung nach Anspruch 3, worin die Cystinbrücken C₆ mit C₁₅ oder C₈ mit C₁₃ verknüpfen.

8. Die Verbindung nach Anspruch 5, worin jedes der 1 - 4 Cysteine unabhängig voneinander durch G, A, S, T, I, V oder L ersetzt ist.

9. Die Verbindung nach Anspruch 1, welche ausgewählt ist aus der Gruppe, bestehend aus 
PG-1: RGGRLCYCRRRFCVCVGR

PG-2: RGGRLCYCRRRFCICV

PG-3: RGGGLCYCRRRFCVCVGR

PG-4: RGGRLCYCRGWICFCVGR
 sowie deren amidierten Formen, entweder in linearer oder cystinverbrückter Form.

10. Die Verbindung nach irgendeinem der Ansprüche 1 bis 9, worin alle Aminosäuren in der D-Konfiguration vorliegen.

11. Eine gereinigte und isolierte Verbindung der Formel:
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1a)
sowie deren N-terminal acylierten, C-terminal amidierten oder veresterten Formen in entweder der linearisierten oder der cystinverbrückten Form,
worin jedes Aₙ unabhängig voneinander an der bezeichneten Position eine Aminosäure ist, und worin die cystinverbrückte Verbindung aus Leukozyten eines Tieres durch ein Verfahren isolierbar ist, welche ein Unterwerfen des Ultrafiltrats eines Lysats der Leukozyten unter chromatographische Trenntechniken, um eine Fraktion zu erhalten, welche Verbindungen der Formel (1a) enthält, und ein Gewinnen der Verbindungen der Formel (1a) umfasst.

12. Ein Peptid in gereinigter und isolierter Form mit antimikrobieller und/oder antiviraler Aktivität, welches durch eine DNA codiert wird, die unter stringenten Bedingungen mit der DNA aus Schweineleukozyten, welche für PG-1, PG-2, PG-3 oder PG-4 codiert, hybridisiert.

13. Eine gereinigte und isolierte DNA, welche unter stringenten Bedingungen mit der DNA aus Schweineleukozyten, welche für PG-1, PG-2, PG-3 oder PG-4 codiert, hybridisiert.

14. Ein rekombinantes Expressionssystem für die Produktion eines Peptids mit der Aminosäuresequenz der Verbindung aus irgendeinem der Ansprüche 1 - 9, wobei das Expressionssystem eine Nukleotidsequenz, die für das Peptid codiert, in funktionsfähiger Verknüpfung mit Steuersequenzen für eine effektive Expression umfasst.

15. Eine rekombinante Wirtszelle, die modifiziert wurde, so dass diese das Expressionssystem aus Anspruch 14 enthält.

16. Ein Verfahren, um ein antimikrobielles oder antivirales Peptid oder ein Zwischenpeptid dafür zu produzieren, wobei das Verfahren ein Kultivieren der modifizierten Wirtszellen aus Anspruch 15 unter Bedingungen, bei welchen das Peptid produziert wird; und
ein Gewinnen des Peptids aus der Kultur umfasst.

17. Das Verfahren nach Anspruch 16, welches weiterhin umfasst: Bewirken von Cystinverknüpfungen des Peptids und/oder Modifizieren des N-Terminus und/oder C-Terminus des Peptids.

18. Eine pharmazeutische Zusammensetzung für eine antimikrobielle oder antivirale Anwendung, welche die Verbindung nach irgendeinem der Ansprüche 1 - 12 in Beimischung mit wenigstens einem pharmazeutisch verträglichen Träger umfasst.

19. Eine Zusammensetzung zur Anwendung auf Pflanzen oder Pflanzenumgebungen zum Verleihen einer Resistenz gegenüber einer mikrobiellen oder viralen Infektion der Pflanzen, welche die Verbindung der Ansprüche 1 - 12 in Beimischung mit wenigstens einem umweltverträglichen Verdünnungsmittel umfasst.

20. Ein Verfahren, um das Wachstum eines Virus oder einer Mikrobe zu verhindern, wobei das Verfahren ein Inkontaktbringen einer Pflanze oder Pflanzenumgebung mit einer Menge der Verbindung nach irgendeinem der Ansprüche 1 - 12, die wirksam ist, um dieses Wachstum zu verhindern, umfasst.

21. Die Verbindung nach irgendeinem der Ansprüche 1 - 12 zur Verwendung in einem Verfahren der Verhinderung des Wachstums eines Virus oder einer Mikrobe in einem Menschen oder einem Tier.

22. Die Verbindung nach Anspruch 21 zur Verwendung als ein Augenpflegeprodukt.

23. Die Verbindung nach irgendeinem der Ansprüche 1 - 12 zur Verwendung bei der Behandlung einer durch Geschlechtsverkehr übertragenen Krankheit.

24. Die Verbindung nach irgendeinem der Ansprüche 1 - 12 zur Verwendung in einem Verfahren, um das Endotoxin gramnegativer Bakterien in einem Menschen oder einem Tier zu inaktivieren.

25. Die Verbindung nach irgendeinem der Ansprüche 1 - 12 zur Verwendung bei der Behandlung eines septischen Schocks.

26. Antikörper, welche spezifisch mit der Verbindung nach irgendeinem der Ansprüche 1 - 12 reagieren.

27. Verwendung der Verbindung nach irgendeinem der Ansprüche 1 - 12 zur Herstellung eines Medikaments zur Verhinderung des Wachstums eines Virus oder einer Mikrobe in einem Menschen oder einem Tier.

## Revendications

1. Composé de formule :
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1)
et ses dérivés à extrémité N-terminale acylée et/ou à extrémité C-terminale amidifiée ou estérifiée,
qui se trouve soit sous forme linéaire, avec les groupes -SH éventuellement stabilisés, soit sous forme à pont(s) cystine,
formule dans laquelle
chacun des A₁ et A₉ est indépendamment un acide aminé basique,
chacun des A₂ et A₃ est indépendamment un petit acide aminé,
chacun des A₅, A₇, A₁₂, A₁₄ et A₁₆ est indépendamment un acide aminé hydrophobe,
chacun des A₄ et A₁₀ est indépendamment un acide aminé basique ou un petit acide aminé,
A₁₁ est un acide aminé basique ou un acide aminé hydrophobe,
A₁₇ est présent ou non, et s'il est présent, c'est un petit acide aminé,
et A₁₈ est présent ou non, et s'il est présent, c'est un acide aminé basique,
ou composé correspondant à une variante de la formule (1) dans laquelle de 1 à 4 des résidus de cystéine sont remplacés chacun par un acide aminé hydrophobe ou par un petit acide aminé, et ses dérivés à extrémité N-terminale acylée et/ou à extrémité C-terminale amidifiée ou estérifiée,
sous réserve que, si ledit composé correspond à la formule 
RGGRLCYCRRRFCVCVGR,
RGGRLCYCRRRFCICV,
RGGGLCYCRRRFCVCVGR,
ou RGGRLCYCRGWICFCVGR,
 sous forme amidifiée et à deux ponts cystine, ce composé est purifié et isolé.

2. Composé conforme à la revendication 1, qui comporte deux ponts cystine
et/ou dans lequel le groupe carboxyle de l'extrémité C-terminale est d'une formule choisie dans l'ensemble formé par -COOH, -COOH salifié, -COOR, -CONH₂, -CONHR et -CONR₂ où chaque symbole R représente indépendamment un groupe hydrocarbyle en C₁₋₆ ;
et/ou dans lequel le groupe amino de l'extrémité N-terminale est de formule -NH₂ ou -NHCOR où R représente un groupe hydrocarbyle en C₁₋₆ ;
et/ou dans lequel chacun des A₁ et A₉ est indépendamment choisi dans l'ensemble formé par R, K et Har ;
et/ou dans lequel chacun des A₂ et A₃ est indépendamment choisi dans l'ensemble formé par G, A, S et T ;
et/ou dans lequel A₄ est R ou G ;
et/ou dans lequel chacun des A₅, A₁₄ et A₁₆ est indépendamment choisi dans l'ensemble formé par I, V, NLe, L et F ;
et/ou dans lequel chacun des A₇ et A₁₂ est indépendamment choisi dans l'ensemble formé par I, V, L, W, Y et F ;
et/ou dans lequel chacun des A₁₀ et A₁₁ est indépendamment R, G ou W.

3. Composé conforme à la revendication 1, qui comporte un seul pont cystine
et/ou dans lequel le groupe carboxyle de l'extrémité C-terminale est d'une formule choisie dans l'ensemble formé par -COOH, -COOH salifié, -COOR, -CONH₂, -CONHR et -CONR₂ où chaque symbole R représente indépendamment un groupe hydrocarbyle en C₁₋₆;
et/ou dans lequel le groupe amino de l'extrémité N-terminale est de formule -NH₂ ou -NHCOR où R représente un groupe hydrocarbyle en C₁₋₆ ;
et/ou dans lequel chacun des A₁ et A₉ est indépendamment choisi dans l'ensemble formé par R, K et Har ;
et/ou dans lequel chacun des A₂ et A₃ est indépendamment choisi dans l'ensemble formé par G, A, S et T ;
et/ou dans lequel A₄ est R ou G ;
et/ou dans lequel chacun des A₅, A₁₄ et A₁₆ est indépendamment choisi dans l'ensemble formé par I, V, NLe, L et F ;
et/ou dans lequel chacun des A₇ et A₁₂ est indépendamment choisi dans l'ensemble formé par I, V, L, W, Y et F ;
et/ou dans lequel chacun des A₁₀ et A₁₁ est indépendamment R, G ou W.

4. Composé conforme à la revendication 1, qui est sous forme linéaire
et/ou dans lequel le groupe carboxyle de l'extrémité C-terminale est d'une formule choisie dans l'ensemble formé par -COOH, -COOH salifié, -COOR, -CONH₂, -CONHR et -CONR₂ où chaque symbole R représente indépendamment un groupe hydrocarbyle en C₁₋₆;
et/ou dans lequel le groupe amino de l'extrémité N-terminale est de formule -NH₂ ou -NHCOR où R représente un groupe hydrocarbyle en C₁₋₆ ;
et/ou dans lequel chacun des A₁ et A₉ est indépendamment choisi dans l'ensemble formé par R, K et Har ;
et/ou dans lequel chacun des A₂ et A₃ est indépendamment choisi dans l'ensemble formé par G, A, S et T ;
et/ou dans lequel A₄ est R ou G ;
et/ou dans lequel chacun des A₅, A₁₄ et A₁₆ est indépendamment choisi dans l'ensemble formé par I, V, NLe, L et F ;
et/ou dans lequel chacun des A₇ et A₁₂ est indépendamment choisi dans l'ensemble formé par I, V, L, W, Y et F ;
et/ou dans lequel chacun des A₁₀ et A₁₁ est indépendamment R, G ou W.

5. Composé conforme à la revendication 1, qui correspond à ladite variante
et/ou dans lequel le groupe carboxyle de l'extrémité C-terminale est d'une formule choisie dans l'ensemble formé par -COOH, -COOH salifié, -COOR, -CONH₂, -CONHR et -CONR₂ où chaque symbole R représente indépendamment un groupe hydrocarbyle en C₁₋₆ ;
et/ou dans lequel le groupe amino de l'extrémité N-terminale est de formule -NH₂ ou -NHCOR où R représente un groupe hydrocarbyle en C₁₋₆ ;
et/ou dans lequel chacun des A₁ et A₉ est indépendamment choisi dans l'ensemble formé par R, K et Har ;
et/ou dans lequel chacun des A₂ et A₃ est indépendamment choisi dans l'ensemble formé par G, A, S et T ;
et/ou dans lequel A₄ est R ou G ;
et/ou dans lequel chacun des A₅, A₁₄ et A₁₆ est indépendamment choisi dans l'ensemble formé par I, V, NLe, L et F ;
et/ou dans lequel chacun des A₇ et A₁₂ est indépendamment choisi dans l'ensemble formé par I, V, L, W, Y et F ;
et/ou dans lequel chacun des A₁₀ et A₁₁ est indépendamment R, G ou W.

6. Composé conforme à la revendication 2, dans lequel lesdits ponts cystine relient C₆ à C₁₅ et C₈ à C₁₃.

7. Composé conforme à la revendication 3, dans lequel ledit pont cystine relie C₆ à C₁₅ ou C₈ à C₁₃.

8. Composé conforme à la revendication 5, dans lequel de 1 à 4 des résidus de cystéine sont remplacés chacun, indépendamment, par G, A, S,T, I, V ou L.

9. Composé conforme à la revendication 1, choisi dans l'ensemble constitué par 
PG-1 : RGGRLCYCRRRFCVCVGR
PG-2 : RGGRLCYCRRRFCICV,
PG-3 : RGGGLCYCRRRFCVCVGR,
PG-4 : RGGRLCYCRGWICFCVGR,
 et leurs dérivés amidifiés, soit sous forme linéaire, soit sous forme à pont(s) cystine.

10. Composé conforme à l'une des revendications 1 à 9, dans lequel tous les acides aminés se trouvent en configuration D.

11. Composé purifié et isolé de formule
A₁-A₂-A₃-A₄-A₅-C₆-A₇-C₈-A₉-A₁₀-A₁₁-A₁₂-C₁₃-A₁₄-C₁₅-A₁₆-(A₁₇-A₁₈) (1a)
et ses dérivés à extrémité N-terminale acylée et/ou à extrémité C-terminale amidifiée ou estérifiée,
qui se trouve soit sous forme linéaire, soit sous forme à pont(s) cystine,
dans lequel chaque Aₙ est un acide aminé situé en la position indiquée,
ledit composé à pont(s) cystine pouvant être isolé chez les leucocytes d'un animal par un procédé qui comporte le fait de soumettre à des opérations de séparation chromatographique le filtrat résultant de l'ultrafiltration d'un lysat desdits leucocytes, pour obtenir une fraction contenant des composés de formule (1a), et le fait de récupérer ces composés de formule (1a).

12. Peptide sous forme purifiée et isolée, présentant une activité antimicrobienne et/ou antivirale, lequel peptide est codé par un ADN qui s'hybride, dans des conditions restrictives, à l'ADN de leucocytes porcins qui code PG-1, PG-2, PG-3 ou PG-4.

13. ADN purifié et isolé, qui s'hybride, dans des conditions restrictives, à l'ADN de leucocytes porcins qui code PG-1, PG-2, PG-3 ou PG-4.

14. Système d'expression recombiné, destiné à la production d'un peptide présentant la séquence d'acides aminés d'un composé conforme à l'une des revendications 1 à 9, lequel système d'expression comporte une séquence de nucléotides codant ledit peptide, opération-nellement liée à des séquences de régulation permettant la réalisation de l'expression.

15. Cellule hôte génétiquement modifiée de telle sorte qu'elle contient un système d'expression conforme à la revendication 14.

16. Procédé de production d'un peptide antiviral ou antimicrobien, ou d'un peptide pouvant servir d'intermédiaire pour obtenir le premier, lequel procédé comporte le fait de cultiver des cellules hôtes génétiquement modifiées conformes à la revendication 15, dans des conditions où ledit peptide est produit, et le fait de récupérer ledit peptide dans la culture.

17. Procédé conforme à la revendication 16, qui comporte en outre le fait d'établir les ponts cystine dudit peptide et/ou le fait de modifier les extrémités N-terminale et/ou C-terminale dudit peptide.

18. Composition pharmaceutique à usage antimicrobien ou antiviral, qui contient un composé conforme à l'une des revendications 1 à 12, mélangé avec au moins un excipient acceptable en pharmacie.

19. Composition à appliquer sur des plantes ou dans l'environnement de plantes, pour conférer à ces plantes une résistance à des infections microbiennes ou virales, qui contient un composé conforme à l'une des revendications 1 à 12, mélangé avec au moins un diluant acceptable du point de vue environnemental.

20. Procédé permettant d'empêcher la prolifération d'un virus ou d'un microbe, lequel procédé comporte le fait de mettre des plantes ou l'environnement de plantes en contact avec un composé conforme à l'une des revendications 1 à 12, utilisé en une quantité qui empêche effectivement ladite prolifération.

21. Composé conforme à l'une des revendications 1 à 12, à employer dans un procédé permettant d'empêcher la prolifération d'un virus ou d'un microbe chez l'homme ou chez un animal.

22. Composé conforme à la revendication 21, à utiliser comme produit de soin pour les yeux.

23. Composé conforme à l'une des revendications 1 à 12, à employer dans le traitement d'une maladie transmissible par voie sexuelle.

24. Composé conforme à l'une des revendications 1 à 12, à employer dans un procédé permettant d'inactiver les endotoxines de bactéries Gram négatif chez l'homme ou chez un animal.

25. Composé conforme à l'une des revendications 1 à 12, à employer dans le traitement d'un choc septique.

26. Anticorps réagissant spécifiquement avec un composé conforme à l'une des revendications 1 à 12.

27. Emploi d'un composé conforme à l'une des revendications 1 à 12 dans la fabrication d'un médicament destiné à empêcher la prolifération d'un virus ou d'un microbe chez l'homme ou chez un animal.
